# EUROPÄISCHE PATENTSCHRIFT

(11) **EP 1 567 143 B1**
(45) Veröffentlichungstag und Bekanntmachung des Hinweises auf die Patenterteilung: **14.08.2013**
(21) Anmeldenummer: 03780002.6
(22) Anmeldetag: 20.11.2003
(51) Int. Cl.: A61K 31/196, A61K 8/44, A61Q 19/00

(54) **ANTHRANILSÄUREAMIDE UND DEREN DERIVATE ALS KOSMETISCHE UND PHARMAZEUTISCHE WIRKSTOFFE**
ANTHRANILIC ACID AMIDES AND THE DERIVATIVES THEREOF AS COSMETIC AND PHARMACEUTICAL AGENTS
AMIDES D'ACIDE ANTHRANILIQUE ET LEURS DERIVES UTILISES COMME PRINCIPES ACTIFS COSMETIQUES ET PHARMACEUTIQUES

(30) Priorität: 25.11.2002 DE 10254872
(43) Veröffentlichungstag der Anmeldung: 31.08.2005
(73) Patentinhaber: Symrise AG, 37603 Holzminden (DE)
(72) Erfinder: SCHMAUS, Gerhard, 37671 Höxter-Bosseborn (DE); JOPPE, Holger, 37586 Dassel (DE); HERRMANN, Martina, 37574 Einbeck (DE); SABATER-LÜNTZEL, Christopher, 37603 Holzminden (DE); VÖSSING, Tobias, 37088 Beverungen (DE)
(74) Vertreter: Eisenführ, Speiser & Partner
(86) Internationale Anmeldenummer: PCT/EP2003/012990
(87) Internationale Veröffentlichungsnummer: WO 2004/047833

(56) Entgegenhaltungen:
- EP-A- 0 391 002
- US-A- 3 940 422
- US-A- 4 070 484
- US-A- 4 486 597
- US-A- 4 587 356
- PATENT ABSTRACTS OF JAPAN Bd. 009, Nr. 309 (C-318), 5. Dezember 1985 (1985-12-05) & JP 60 146856 A (KISSEI YAKUHIN KOGYO KK), 2. August 1985 (1985-08-02)
- PATENT ABSTRACTS OF JAPAN Bd. 006, Nr. 110 (C-109), 22. Juni 1982 (1982-06-22) & JP 57 038759 A (KISSEI PHARMACEUT CO LTD), 3. März 1982 (1982-03-03)
- ISHIHARA A ET AL: "Induction of hydroxyanthranilate hydroxycinnamoyl transferase activity by oligo-N-acetylchitooligosaccharides in oats" PHYTOCHEMISTRY, PERGAMON PRESS, GB, Bd. 47, Nr. 6, 1998, Seiten 969-974, XP004293820 ISSN: 0031-9422
- DATABASE CHEMABS [Online] CHEMICAL ABSTRACTS SERVICE, COLUMBUS, OHIO, US; MA, JUNJIANG ET AL: "Effects of N-(3',4',5'-trimethoxycinnamoyl) ortho -aminobenzoic acid on antigen-induced contraction of guinea pig ileum and the degranulation of an histamine release from mast cells" XP002285836 gefunden im STN Database accession no. 1993:73312 & JOURNAL OF CHINESE PHARMACEUTICAL SCIENCES , 1(1), 41-5 CODEN: JCHSE4; ISSN: 1003-1057, 1992,
- DATABASE CHEMABS [Online] CHEMICAL ABSTRACTS SERVICE, COLUMBUS, OHIO, US; YIN, K. ET AL: "Interaction between terbutaline and ethyl 2-(4'-carboxybenzamido)-4- propionamidobenzoate sodium salt in tracheal smooth muscle relaxation of guinea pig" XP002285837 gefunden im STN Database accession no. 1990:417725 & ARZNEIMITTEL-FORSCHUNG , 40(4), 446-9 CODEN: ARZNAD; ISSN: 0004-4172, 1990,
- GHELARDONI, MARIO ET AL: "Quaternary salts of substituted 2-aminoethyl N-benzoylaminobenzoate. New class of smooth muscle relaxant agents" JOURNAL OF MEDICINAL CHEMISTRY , 16(9), 1063-5 CODEN: JMCMAR; ISSN: 0022-2623, 1973, XP002285835

## Beschreibung

Die vorliegende Erfindung betrifft bestimmte Verwendungen von Verbindungen der Formel 1 oder von Mischungen aus zwei oder mehr unterschiedlichen Verbindungen der Formel 1.

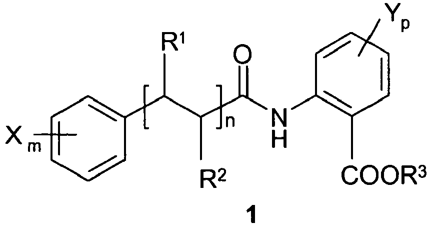

Die Erfindung betrifft zudem ausgewählte Verbindungen der Formel 1, die bislang nicht bekannt waren.

Ein erster Aspekt der vorliegenden Erfindung betrifft eine kosmetische Verwendung einer Verbindung der Formel 1 oder einer Mischung aus zwei oder mehr unterschiedlichen Verbindungen der Formel 1 zur nicht therapeutischen Behandlung von oder Vorbeugung gegen Juckreiz (Pruritus), Hautrötung oder Quaddelbildung.

Hierbei gilt für die Verbindung der Formel 1 bzw. jede Verbindung der Formel 1 in der Mischung:
m = 0, 1, 2 oder 3,
p = 0, 1 oder 2,
n=1,
mit der Maßgabe, dass die Summe p + m > 0 ist,
wobei R¹ und R² jeweils H bedeuten;
wobei bei m = 1, 2 oder 3 jedes X, unabhängig von den anderen, OH, OAlkyl oder OAcyl bedeutet,
wobei bei p = 1 oder 2 jedes Y, unabhängig von den anderen, OH, OAlkyl oder OAcyl bedeutet,
mit der Maßgabe, dass mindestens einmal X oder Y aus der Gruppe gewählt ist, die aus OH und OAcyl besteht;
R³ = H oder Alkyl (insbesondere -CH₃ sowie lineare oder verzweigte Alkylketten mit 2 bis 30 C-Atomen)
R³ = H steht dabei auch für die korrespondierenden pharmazeutischen akzeptablen Sätze.

Eine Verbindung der Formel 1 kann dabei in Form eines beliebigen Isomeren oder Isomerengemisches vorliegen.

Für X oder Y = OAcyl gilt vorzugsweise: Acyl = CO-R mit R = -CH₃, linear oder verzweigter Alkylrest mit 2-30 C-Atomen.

Mastzellen spielen eine wesentliche Rolle bei allergischen und inflammatorischen Prozessen, da sie nach entsprechender Stimulation Mediatoren wie Histamin freisetzen, die in hohem Maße für Symptome wie Juckreiz (Pruritus), Schmerz- oder Rötungsreaktionen verantwortlich sind. Zahllose Untersuchungen konnten zeigen, dass das Neuropeptid Substanz P, welches von Nervenenden in der Haut freigesetzt wird, eine Degranulation von Mastzellen herbeiführt. Somit kann Substanz P als eines der wichtigsten Bindeglieder zwischen dem peripheren Nervensystem und Symptomen wie Entzündungsprozessen, Juckreiz und Schmerz betrachtet werden.

In der pharmazeutischen und kosmetischen Industrie besteht ein ständiger Bedarf an Inhibitoren der Freisetzung von Histamin. Die unkontrollierte Freisetzung hoher Histamin-Konzentrationen geht einher mit Symptomen wie Juckreiz, Schmerz oder Rötungsreaktionen. Eine eindeutige Korrelation zwischen Histamin-Freisetzung und starkem Juckreiz wurde vor allem bei der Urtikaria (Nesselsucht) festgestellt. Der Name Urtikaria leitet sich historisch von den Symptomen ab, welche nach Kontakt der Haut mit Brennesseln (lat. Name: *Urtica dioica* L.) zu beobachten sind (Juckreiz, Brennen, Quaddelbildung). In diesem speziellen Fall wirkt u.a. bereits in der Brennessel enthaltenes Histamin, welches in speziellen Sekretionsorganen gespeichert und nach Kontakt mit der Haut über die sog. Brennhaare, die die Form einer Injektionsnadel besitzen, in die Haut injiziert wird. In Folge kommt es zu Rötung, Juckreiz und Quaddelbildung.

Diversen Formen der Urtikaria ist eines gemeinsam, nämlich die Aktivierung eines speziellen Zelltyps, der sogenannten Mastzellen. Mastzellen könnte man auch als "Feuerwehr" oder "Grenzpolizei" des menschlichen Körpers bezeichnen. Sie sind besonders häufig dort anzutreffen, wo wir mit unserer Umwelt in unmittelbarem Kontakt stehen, also neben der Haut auch in den Schleimhäuten des Magen-Darm-Traktes und der Atemwege. Die Aktivierung der Mastzellen durch unterschiedliche Mediatoren (Immunoglobuline wie IgE oder Neuropeptide wie Substanz P) kann mit starken Entzündungsreaktionen, Juckreiz und stark allergischen Reaktionen bis hin zum anaphylaktischen Schock einhergehen. Zu diesem Zweck stellen die Mastzellen eine große Vielzahl von Produkten her, unter anderem Histamin. Diese Produkte werden von den Zellen in Vorratsbehältern, den sogenannten Granula, gespeichert und bei einer Aktivierung in großer Menge in die Haut freigesetzt. Dies wiederum führt dazu - und hier kommt dem Histamin eine besonders wichtige Rolle zu -, dass die Blutgefäße an der betroffenen Hautstelle "undicht" werden und Blutbestandteile (hauptsächlich Flüssigkeit) in das Gewebe dringen. Als Ergebnis entsteht eine Quaddel. Darüberhinaus kommt es zur Weitung der Blutgefäße (Vasodilatation). In Folge werden die entsprechenden Hautregionen stärker durchblutet, was zu Rötung führt. Die Entstehung des Juckreizes erklärt man sich folgendermaßen: Zum einen führt das Freisetzen von Histamin aus Mastzellen unmittelbar zu Juckreiz. Zum anderen stimulieren Histamin und andere Mastzell-Produkte Nervenfasern in der Haut. Diese Stimulation hat nun zur Folge, dass die Nervenfasern Juckreiz-auslösende Substanzen (sogenannte Neuropeptide) freisetzen. Diese Neuropeptide (z.B. Substanz P) sind wiederum gute Mastzell-Aktivierer, so dass die Stimulation von Nerven durch Mastzellen eine Stimulation von Mastzellen durch Nerven zur Folge hat. Mastzellen sind in der Haut und in Schleimhäuten bevorzugt in der unmittelbaren Nähe von Gefäßen und Nerven lokalisiert. Kein Wunder also, dass die Verständigung zwischen den "Nachbarn" Mastzelle, Gefäßzelle und Nervenfaser hervorragend funktioniert.

Juckreiz, der mitunter auf die Freisetzung hoher Histamin-Konzentrationen aus Mastzellen zurückzuführen ist, kann bei verschiedenen Krankheitszuständen auftreten. Hierzu zählen im wesentlichen allergische Hautreaktionen (Lebensmittelallergien, Chemikalien), Pruritus (Nesselsucht), Kontakt mit Pflanzen (z.B. Brennnessel), Insektenstiche, Schuppenflechte, Infektionen sowie leichte Verbrennungen, abheilende Verletzungen physikalische Reize wie Wärme oder mechanische Reibung und Nickelallergie.

Überraschenderweise hat sich in umfangreichen eigenen Untersuchungen gezeigt, dass die oben genannten Verbindungen der Formel 1 und Mischungen aus zwei oder mehr unterschiedlichen Verbindungen der Formel 1 hervorragend geeignet sind, die Substanz P induzierte Freisetzung von Histamin aus Mastzellen zu inhibieren. Ausgewählte Untersuchungsergebnisse sind weiter unten in Form von Beispielen und Tabellen zusammengefasst.

Unter den erfindungsgemäß verwendbaren Verbindungen der Formel 1 sind bestimmte Untergruppen und Einzelsubstanzen besonders zur Inhibierung der Substanz P induzierten Freisetzung von Histamin aus Mastzellen geeignet. Diese bevorzugten Untergruppen und Einzelsubstanzen lassen sich den abhängigen Ansprüchen, den nachfolgenden Beispielen und den zugehörigen Tabellen entnehmen.

Im Rahmen der vorliegenden Erfindung wurde überraschenderweise festgestellt, dass bestimmte Verbindungen der Formel 1 oder Mischungen aus zwei oder mehr unterschiedlichen Verbindungen der Formel 1 nicht nur zur Inhibierung der Substanz P induzierten Freisetzung von Histamin aus Mastzellen eingesetzt werden können, sondern allgemeiner zur Behandlung von oder Vorbeugung gegen Juckreiz (Pruritus), Hautrötung, Quaddelbildung und/oder allergische Hautreaktionen (sowie zur Herstellung entsprechender Arzneimittel).

Generell gilt zwar, dass sich die oben als geeignet zur erfindungsgemäßen Inhibierung der Substanz P induzierten Freisetzung von Histamin aus Mastzellen angegebenen Verbindungen der Formel 1 auch zu dem allgemeineren Zweck der Behandlung von oder Vorbeugung gegen Juckreiz, Hautrötung, Quaddelbildung oder allergische Hautreaktionen verwenden lassen.

Aber auch insoweit sind bestimmte Verbindungen der Formel 1 bevorzugt, die sich den abhängigen Ansprüchen, den Beispielen und den zugehörigen Tabellen entnehmen lassen.

In diesem Zusammenhang sei darauf hingewiesen, dass Juckreiz nicht nur im Zusammenhang steht mit den oben angegebenen Mechanismen sondern auch mit trockener Haut, Altershaut, mechanisch, chemisch oder durch Sonnenlicht gestresster Haut in Verbindung gebracht wird. Weiterhin können auch psychologische Faktoren wie Angst oder Stress Juckreiz hervorrufen. Seifen, Putz- und Lösungsmittel, die die Haut stark belasten, können zu einem gestörten Hydrolipidfilm führen und in Folge Juckreizreaktionen hervorrufen. Auch beim Heilungsprozess von Verletzungen oder Verbrennungen (z.B. nach der Rasur oder nach Sonnenbrand) kann es zu Juckreiz kommen, so dass die Anwendung Juckreiz vermeidender oder lindernder kosmetischer Formulierungen auch hier zum Erhalt eines optimalen physiologischen Zustands der Haut beitragen kann. Hinzu kommt das weite Feld der Kosmetikprodukte, die bei alternder Haut zum Einsatz gelangen. Obgleich die zugrundeliegenden biochemischen Vorgänge von Juckreiz- und Rötungsreaktionen nicht im Detail geklärt sind, gibt es Erkenntnisse, dass das Histamin auch hierbei eine wesentliche Rolle spielt.

Es ist deshalb leicht nachvollziehbar, dass in der Pharmazie und in der Kosmetikindustrie ein großes Interesse am Auffinden von Wirkstoffen besteht, die die Freisetzung von Histamin aus Mastzellen inhibieren und/oder zur Behandlung oder Vorbeugung gegen Juckreiz, Hautrötung, Quaddelbildung oder allergische Hautreaktionen einsetzbar sind.

Die erfindungsgemäßen Verbindungen der Formel 1 oder die Mischungen aus zwei oder mehr unterschiedlichen Verbindungen der Formel 1 können als Bestandteil eines gegebenenfalls nachbehandelten Pflanzenextraktes vorliegen. Insbesondere kann es sich bei dem gegebenenfalls nachbehandelten Pflanzenextrakt um einen Extrakt aus Pflanzen der Gattung Avena, Dianthus, Silene oder Melandrium handeln, insbesondere um Haferextrakte oder Nelkenextrakte.

Falls ein Pflanzenextrakt nachbehandelt wird, wird hierdurch vorzugsweise dafür gesorgt, dass im Vergleich mit dem nicht nachbehandelten Extrakt der Anteil an einer oder mehreren Verbindungen der Formel 1 relativ zu dem Anteil sonstiger extrahierter Verbindungen erhöht ist.

Wenngleich dies nicht in allen Fällen erforderlich ist, so ist es doch in manchen Fällen vorteilhaft, eine isolierte und aufgereinigte Verbindung der Formel 1 oder eine isolierte und aufgereinigte Mischung aus zwei oder mehr unterschiedlichen Verbindungen der Formel 1 zu den genannten Verwendungszwecken einzusetzen. In der Isolierung und Aufreinigung von Verbindungen der Formel 1 oder entsprechenden Mischungen aus einem Pflanzenextrakt ist eine Nachbehandlung zu sehen, bei der der Anteil an Verbindungen der Formel 1 relativ zum Anteil sonstiger extrahierter Verbindungen erhöht wird.

Die vorliegende Erfindung betrifft schließlich gemäß einem weiteren Aspekt auch ausgewählte Verbindungen der Formel 1, die zu den genannten Zwecken eingesetzt werden können und bislang nicht bekannt waren. Diese neuen und erfindungsgemäßen Verbindungen, nämlich die Verbindungen 5, 8 und 11 lassen sich der Tabelle 1a entnehmen.

Zum Stand der Technik sei das Folgende angemerkt:
Zur Linderung von schwach ausgeprägtem Juckreiz sind u.a. bereits folgende therapeutische Maßnahmen bekannt: Medizinische Ölbäder mit Sojabohnenöl (Balneum-Hermal), Paraffinum liquidum (Oleatum-Fett) oder γ-Linolensäure (Linola-Fett-Ölbad), Körpercremes (W/O-Emulsionen als Lotionen) wie z. B. Bepanthenol Roche Lotio F oder Eucerin cum aqua, Körperöle mit Mandel- oder Jojobalöl und Präparate mit Feuchthaltefaktoren wie z.B. Harnstoff oder Salicylsäure. Bei stark ausgeprägten Exanthemen/Quaddeln werden z.B. steroidhaltige Externa appliziert.

Zu Wirkstoffen, deren Juckreiz lindernde Wirksamkeit auf einer gezielten Inhibition der mediatorinduzierten Freisetzung von Histamin aus Mastzellen beruht zählen zum Beispiel Dinatriumcromogycat, Verapamil, Ketotifen oder Tranilast. Bei der unter dem Handelsnamen Tranilast bekannten Substanz handelt es sich um N-(3,4-Dimethoxy-cinnamoyl)anthranilsäure, ein natürlich in der chinesischen Heilpflanze *Nandina domestica* vorkommendes Anthranilsäureamid, das allerdings nicht unter die Formel 1 fällt.

Über Einsatzmöglichkeiten des Tranilast bei allergischen Reaktionen wie Bronchialasthma, allergischer Rhinitis, allergischer Conjunctivitis, Lebensmittelallergien, Urtikaria oder atopischer Dermatitis wird in verschiedenen Patenten und Veröffentlichungen berichtet (DE 2402398; EP 0074725; US 4070484; H.Shioda et al., Allergy 34, 213-119 (1979); M.Kojima et al., Oyo Yakuri 28(4), 623-628 (1984), Azuma et al., Br. J. Pharmacol. Vol.58, p.483-488, (1976); Koda et al., Int. Archs. Allergy appl. Immun. Vol.77, p. 244-245, (1985); Komatsu et al., Japan J. of Pharmacol. Vol.46, 43-51,(1988); Hachisuka et al, Arch. Dermatol. Res. Vol. 280, p.158-162, (1988).

Wie Komatsu et al berichten, inhibiert Tranilast die Antigen- (DNP-ascaris; monoklonale IgE-Antikörper mit 2,4-Dinitrophenyl-Spezifität) induzierte Freisetzung von Histamin aus Mastzellen im Konzentrationsbereich von 10-³ bis 10⁻⁵M. Eine 50%-ige Inhibition des Histamin-Release konnte bei einer Konzentration von ca. 10⁻⁴ M erzielt werden. Der Einfluss anderer Anthranilsäureamide auf die Inhibition der Freisetzung von Histamin aus Mastzellen wurde in der Veröffentlichung hingegen nicht beschrieben.

Hachisuka et al. (Arch. Dermatol. Res. Vol.280, p.158-162; 1988) untersuchten den Einfluss verschiedener Wirkstoffe auf die Inhibition der Substanz P induzierten Freisetzung von Histamin aus Mastzellen. Verglichen wurde hier die Wirksamkeit von Dinatriumcromogylcat, Ketotifen und Tranilast. Eine 50%-ige Inhibition des Substanz P induzierten Histamin-Release konnte erst bei einer relativ hohen Konzentration von ca. 10⁻³ M erzielt werden. Bei einem Molekulargewicht des Tranilast von 327 entspricht 10⁻³M einer Konzentration von 327µg/ml bzw. 327 ppm. Der Wert für DSCG (Dinatriumcromoglycat), ein bekanntes Antiallergikum, lag in einem vergleichbaren Konzentrationsbereich (41% Inhibition bei 10⁻³ M). Andere Anthranilsäureamide wurden auch im Rahmen dieser Forschungsarbeiten nicht näher untersucht.

Kojima Masami et al. (Oyo Jakuri, 28(4), p. 623-28; 1984) beschreiben die anti-allergische Wirkung anderer Anthranilsäureamide, wie zum Beispiel ein in Position 4 demethyliertes Tranilast (N-(3-methoxy,4-hydroxycinnamoyl)anthranilsäure), welches allerdings nur einen gleich stark inhibierenden Effekt wie Tranilast aufweisen soll.

In der US 4,070,484 wird beschrieben, dass die orale Applikation monosubstituierter N-(4-Hydroxycinnamoyl)anthranilsäure bei einer Dosis von 200 mg/kg eine antigen-induzierte Zerstörung von Ratten-Mastzellen zu 36,7% inhibiert, wohingegen das entsprechende (dimethylierte) Tranilast (N-(3,4-Dimethoxycinnamoyl)anthranilsäure) unter identischen VersuchsBedingungen eine deutlich höhere, nämlich 46,1%-ige Inhibitionsrate aufweist. Vergleichende Werte speziell zur Inhibition der Substanz P induzierten Freisetzung von Histamin aus Mastzellen werden in der Schrift nicht gegeben.

Anthranilsäureamide sind als Inhaltsstoffe bestimmter Pflanzen, wie zum Beispiel Hafer *(Avena sativa)* oder Nelken *(Dianthus sp.),* beschrieben.

Bei den Avenanthramiden des Hafer handelt es sich um Säureamide bestehend aus unsubstituierten oder substituierten Anthranilsäure-Partialstukturen und unsubstituierten oder substituierten Zimtsäure-Partialstrukturen, wohingegen die Säureamide in Nelken-Arten im wesentlichen aus Anthranilsäure- und Benzoesäure-Partialstrukturen zusammengesetzt sind. Pflanzenphysiologische Untersuchungen zeigten, dass sowohl die sogenannten Avenanthramide des Hafers als auch die sogenannten Dianthramide in Nelken als Phytoalexine wirken und von der Pflanze nach mikrobiellem Befall im Rahmen eines Abwehrmechanismus gebildet werden.

Die Verwendung von Haferextrakten zur Linderung von Juckreiz ist in der Volksmedizin seit langen bekannt und wird in folgenden Schriften erwähnt: Hagers Handbuch der Pharmazeutischen Praxis, Bd. 4, Hrsg. R. Hänsel, K. Keller, H. Rimpler, G. Schneider, Springer Verlag, Berlin, 1992, p. 437-446; US Federal Register October 3/1989; 54, 190 proposed rules, pp 40808-40811; Bundesanzeiger Nr. 193, 15.10.1987). Die Verwendung spezieller Haferextrakt-Fraktionen mit angereichertem Gehalt an Anthranilsäureamiden zur Linderung von Juckreiz und Haut-rötung wurde in einer neueren Veröffentlichung beschrieben (J.Vollhardt et al., Proceedings of the XXIst IFSCC International Congress, Berlin, Sept. 11-14, 2000, p. 395; Verlag für Chemische Industrie, H. Ziolkowsky GmbH Augsburg Germany). In dieser Publikation wird jedoch nicht berichtet, welches oder welche Anthranilsäureamide aus der mehr als 30 Substanzen umfassenden Gruppe der sogenannten Hafer-Avenanthramide an der Linderung von Rötungsreaktionen und Juckreiz beteiligt sind.

Der vorliegenden Erfindung liegen umfangreiche Arbeiten insbesondere zu folgenden Aspekten zugrunde:
- Struktur-Aktivitäts-Betrachtungen zur Identifikation von Anthranilsäureamiden mit rötungs- und juckreizlindernder Wirkung.
- Herstellung von Pflanzenextrakten mit hohem Gehalt an spezifischen, hochwirksamen Anthranilsäureamiden, insbesondere Herstellung spezifischer Hafer- und Nelkenextrakt-Fraktionen, sowie aufgereinigter Wirkstoffisolate insbesondere aus Hafer und/oder Nelke.
- Synthese von Anthranilsäureamiden mit Juckreiz-lindernder Wirkung.

Durch die genannten Arbeiten sollten besonders wirksame Verbindungen in vorzugsweise hochreiner Form verfügbar gemacht werden, so dass sie frei von eventuell toxikologisch oder dermatologisch kritischen Nebenbestandteilen (Synthese Nebenprodukten oder Pflanzenextrakt-Nebenbestandteilen) in pharmazeutischen oder kosmetischen Produkten zum Einsatz gelangen können.

Generell war hierbei zu beachten, dass die in kosmetischen und/oder pharmazeutischen Produkten zu verwendenden Substanzen im Wirksamkeits-relevanten Konzentrationsbereich
- toxikologisch unbedenklich,
- gut hautverträglich,
- stabil (insbesondere in den üblichen kosmetischen und/oder pharmazeutischen Formulierungen),
- vorzugsweise geruchlos und
- preiswert herstellbar (d.h. unter Einsatz von Standardverfahren und/oder ausgehend von Standardprekursoren)
sein sollten.

Bevorzugte Ausgestaltungen der erfindungsgemäßen Verwendungen sowie erfindungsgemäße Verbindungen lassen sich den nachfolgenden Beispielen und den zugehörigen Tabellen entnehmen:

### 1. Synthese von Anthranilsäureamiden der Formel 1

### Beispiel 1

Synthese von kern-unsubstituierten oder -substituierten Cinnamoylanthranilsäure-Derivaten am Beispiel von N-(4-Hydroxycinnamoyl) anthranilsäure (Avenanthramid D, **10;** vgl. Tabelle 1 a, nicht erfindungsgemäß) 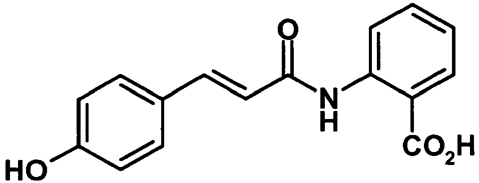 Avenanthramid D **(10)**

15 g (91 mmol) 4-Hydroxyzimtsäure, 50 ml Acetanhydrid und 0.5 ml Pyridin werden 20h bei Raumtemperatur gerührt. Das Gemisch wird in Eiswasser gegossen, die ausgefallene 4-Acetoxyzimtsäure abgetrennt und getrocknet (18 g). 15 g (77 mmol) 4-Acetoxyzimtsäure werden vorgelegt und 15 g (100 mmol) Thionylchlorid zugetropft. Anschließend wird 1 h unter Rückfluss gerührt, das überschüssige Thionylchlorid destillativ abgetrennt und das Säurechlorid mit 50 ml Toluol versetzt. Eine Lösung von 7 g (51 mmol) Anthranilsäure in 70 ml Pyridin wird zugegeben und das Reaktionsgemisch 2 h bei 90 °C gerührt. Nach Abkühlen wird die Reaktionsmischung in Eiswasser gegossen, mit Ethylacetat extrahiert, die organische Phase mit Wasser neutralgewaschen und im Vakuum das Lösungsmittel entfernt. Der Rückstand wird mit 300 g 10%iger Natronlauge versetzt und 1-2 h unter Rückfluss gerührt. Nach Abkühlen wird mit konz. Salzsäure angesäuert, mit Ethylacetat extrahiert, die organische Phase mit Wasser neutralgewaschen und im Vakuum vom Lösungsmittel befreit. Das Rohprodukt (23 g) wird durch wiederholtes Lösen unter Erhitzen in Ethanol, Abkühlen und Ausfällen durch Zugabe von Wasser umkristallisiert und durch RP-18 Mitteldruckchromatographie (Säule: YMC ODS-AQ, Eluent: Methanol/Wasser 50:50 + 0.5 ml Essigsäure/I, λ 280 nm) weiter aufgereinigt (Ausbeute: 1.1 g **10**, Reinheit: 95%).

Spektroskopische Daten: ¹H-NMR (300 MHz, D₆-Aceton): 8.91 (1 H, dd, J = 1.2 und 8.5 Hz), 8.14 (1H, dd, J = 1.7 und 8.1 Hz), 7.67 (1H, d, J = 15.4 Hz), 7.61 (1 H, m), 7.60 (2 H, d, J = 8.7 Hz), 7.16 (1 H, m), 6.92 (2 H, d, J = 8.7 Hz), 6.64 (1 H, d, J = 15.4 Hz). - ¹³C-NMR (75.5 MHz, D₆-Aceton): 170.5 (s), 165.3 (s), 160.2 (s), 143.3 (s), 142.7 (d), 135.2 (d), 132.2 (d), 130.8 (2C, d), 127.3 (s), 123.0 (d), 120.9 (d), 119.8 (d), 116.6 (2C, d), 115.8 (s).

### Beispiel 2

Katalytische Hydrierung von kern-unsubstituierten oder -substituierten Cinnamoylanthranilsäure-Derivaten zur entsprechenden Dihydro-Verbindung (**8**, Tabelle 1 a, erfindungsgemäß) am Beispiel von N-(4-Hydroxycinnamoyl)anthranilsäure (Avenanthramid D, **10**)

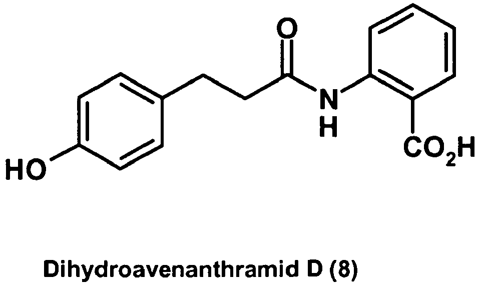

140 mg N-(4-Hydroxycinnamoyl)anthranilsäure (Avenanthramid D, **10**) werden in 20 ml Ethanol aufgenommen und in Gegenwart von Palladium (5% auf Aktivkohle) mit Wasserstoff quantitativ hydriert.

Spektroskopische Daten: ¹H-NMR (300 MHz, D₆-Aceton): 8.68 (1 H, dd, J = 0.8 und 8.4 Hz), 8.08 (1 H, dd, J = 1.5 und 7.8 Hz), 7.59 (1 H, m), 7.15 (1 H, m), 7.12 (2 H, d, J = 8.5 Hz), 6.77 (2 H, d, J = 8.5 Hz), 2.95 (2 H, t, J = 7.6 Hz), 2.75 (2 H, t, J = 7.6 Hz).

### 2. Gewinnung von Anthranilsäureamid-Fraktionen aus Pflanzen

### Beispiel 3

### Gewinnung eines Anthranilsäureamid-haltigen Extraktes aus Haferstroh (Avena sativa)

9 kg Haferstroh werden mit 143 kg Ethanol/Wasser 7:3 (w/w) versetzt und bei Raumtemperatur 3 Tage mazeriert. Nach Filtration wird der Extrakt im Vakuum bis zur Wasserphase eingeengt (17.4 kg, Trockensubstanzgehalt: 2.5%, Σ Avenanthramide A, B & C (nicht erfindungsgemäß) in Trockenextrakt: 0.093%).

Die wässrige Lösung wird portionsweise (2 kg) mit Amberlite XAD-16 (270 g) ausgerührt. Das Adsorberharz wird über eine Fritte abgetrennt, mit Wasser gewaschen und mit Methanol/Wasser 1:1 (v/v) eluiert. Die vereinigten Eluate werden im Vakuum vom Lösungsmittel befreit. Trockenextrakt: 8.5 g, Σ Avenanthramide A, B & C. 1.2%.

Dieser Trockenextrakt wird in Ethanol/Wasser 1:1 (v:v) aufgenommen und durch Verdünnnen mit Ethanol/Wasser 1:1 (v:v) auf einen Avenanthramidgehalt Σ A, B & C von 500 ppm eingestellt.

### 3. Wirksamkeitsstudien zur Inhibition der Substanz P induzierten Freisetzung von Histamin aus Mastzellen durch Anthranilsäureamide der allg. Formel 1.

### 3.1. Synthetische Anthranilsäureamide

### Testprodukte

Stammlösungen : Avenanthramide 2 bis 19 (Strukturformeln: siehe Tabelle 1 a) bzw. Dianthramide 20-29 (Strukturformeln: siehe Tabelle 1 b); Konzentration: 1 % in Ethanol; Lagerung vor Benutzung bei 4°C)

### Reagenzien

Substance P : Fa. BACHEM.

### Testdesign:

Mastzellen aus dem Bauchfellgewebe von Ratten wurden durch Zentrifugation an Metrizamid isoliert und mit Substance P (10µM) stimuliert. Als Positivkontrolle diente Calciumchlorid.

### Probenvorbereitung: Test und Referenzsubstanzen:

Die synthetisch hergestellten Avenanthramide und Dianthramide mit den Formeln 2 bis 29 (vgl. Tabellen 1a/1b) wurden mit Puffermedium bis zu den endgültigen Konzentrationen (0.5 ; 5 und 50 ppm, siehe Tabellen 1a/1b), entsprechend einer Ethanol-Konzentration von 0.5 ; 0.05 bzw. 0.005 % (v/v) verdünnt. Calciumchlorid, welches als Positivkontrolle diente, wurde ebenfalls mit Puffermedium verdünnt, sodass Lösungen im Konzentrationsbereich von 10⁻⁸ bis 10⁻² M verfügbar waren.

### Inkubationsprotokoll:

Testsystem : Die endgültigen Testlösungen wurden in Anwesenheit von Substanz P und der Referenzsubstanz (carl₂) bzw. der jeweils zu testenden Probe für 2 Minuten inkubiert (Inkubationstemperatur: 37°C). Als Nullprobe dienten Zellen die in Abwesenheit von Substanz P und Referenz- bzw. Probensubstanz inkubiert wurden.

### Bestimmung des Gesamthistanmingehalts nach Zelllyse:

Zunächst wurden die Reaktionslösungen durch Zentrifugation von Zellbestandteilen befreit. Anschließend wurde das im Überstand befindliche Histamin mit OPT derivatisiert und die Fluoreszenz der Lösungen photometrisch (Fluorometer Cytofluor 2350) bestimmt.

### Ergebnis:

Calciumchlorid inhibierte konzentrationsabhängig die Substanz P induzierte Freisetzung von Histamin aus Mastzellen. Ein 50%-ige Inhibition konnte bei einer Calciumchlorid-Konzentration von 639 µM erreicht werden. Dies entspricht dem zu erwartenden Wert und validiert somit das zugrundeliegende Testdesign

Die Werte für die verschiedenen Avenanthramide bzw. Dianthramide sind in Tabelle 1a/1b als %-Inhibition bezogen auf den Histamingehalt mit Substanz P stimulierter Zellen dargestellt.

### 3.2. Anthranilsäureamid-Fraktion aus Hafer-Samen im direkten Vergleich zu einem Rekonstitut bestehend aus identischen Mengenverhältnissen an synthetisch hergestellten Hafer-Anthranilsäureamiden

### Testprodukte

Stammlösungen : GS-101100-A und GS-101100-B (Zusammensetzung der Proben: siehe Tabelle 2; Konzentration: 500 ppm Σ Avenanthramide A, B und C in Glycerin; Lagerung vor Benutzung bei 4°C)

### Reagenzien

Substance P : Fa. BACHEM.

### Testdesign:

Mastzellen aus dem Bauchfellgewebe von Ratten wurden durch Zentrifugation an Metrizamid isoliert und mit Substance P (10µM) stimuliert. Als Positivkontrolle diente Calciumchlorid.

### Probenvorbereitung: Test und Referenzsubstanzen:

Die Proben GS-101100-A und GS-101100-B wurden mit Puffermedium bis zu den endgültigen Konzentrationen (0.5 ; 5 und 50 ppm, siehe Tabelle 2), entsprechend einer Ethanol-Konzentration von 0.1 ; 1 bzw. 10 % (v/v) verdünnt. Calciumchlorid, welches als Positivkontrolle diente, wurde ebenfalls mit Puffermedium verdünnt, sodass Lösungen im Konzentrationsbereich von 10⁻⁵ und 10⁻² M verfügbar waren.

### Inkubationsprotokoll:

Testsystem : Die endgültigen Testlösungen wurde in Anwesenheit von Substanz P und der Referenzsubstanz (CaCl₂) bzw. der jeweils zu testenden Probe für 2 Minuten inkubiert (Inkubationstemperatur: 37°C). Als Nullprobe dienten Zellen die in Abwesenheit von Substanz P und Referenz- bzw. Probensubstanz inkubiert wurden.

### Bestimmung des Gesamthistamingehalts nach Zelllyse:

Zunächst wurden die Reaktionslösungen durch Zentrifugation von Zellbestandteilen befreit. Anschließend wurde das im Überstand befindliche Histamin mit OPT derivatisiert und die Fluoreszenz der Lösungen photometrtisch (Fluorometer Cytofluor 2350) bestimmt.

### Ergebnis:

Calciumchlorid inhibierte konzentrationsabhängig die Substanz P induzierte Freisetzung von Histamin aus Mastzellen. Ein 50%-ige Inhibition konnte innerhalb dieser Testreihe bei einer Calciumchlorid-Konzentration von 432µM erreicht werden. Dies entspricht dem zu erwartenden Wert und validiert somit das zugrundeliegende Testdesign

Die Werte für die verschiendenen Avenanthramide sind in Tabelle 2 als %-Inhibition bezogen auf den Histamingehalt mit Substanz P stimulierter Zellen ohne Zusatz von Inhibitoren dargestellt.

### 4. Ergebnisse und Struktur-Aktivitätsbetrachtungen

### 4.1. Synthetische Anthranilsäureamide

Ergebnisse von Untersuchungen ausgewählter hochreiner Anthranilsäureamide mit Zimtsäure-bzw. Dihydrozimtsäure-Partialstruktur (Avenanthramide), zeigen, dass in Abhängigkeit vom Substitutionsmuster signifikante Unterschiede in der Wirksamkeit zu verzeichnen sind. Die stärkste Wirksamkeit, d.h. eine komplette Inhibition der Histamin-Freisetzung erzielt man mit Substanzen der Formeln **2** und **3** (nicht erfindungsgemäß, vgl. Tabelle 1 a), die vicinale Hydroxyl-Funktionen in den Positionen 3 und 4 des Zimtsäure-/Dihydrozimtsäure-Teils besitzen. Weitere Hydroxyl-Funktionen im Anthranilsäure-Teil führen hier hingegen zu keiner zusätzlichen Wirksamkeitserhöhung. Dies zeigen die nahezu identischen Inhibitionsdaten für Substanz **2** (7%, 80% und 108% bei einer Wirkstoffkonzentration von 0,5; 5 bzw. 50 ppm, vgl. Tabelle 1a) und Substanz **3** (11%, 62% und 106% bei einer Wirkstoffkonzentration von 0,5; 5 bzw. 50 ppm, vgl. Tabelle 1 a). Eine Inhibition der Histamin-Freisetzung >50% ist somit bereits bei einer Dosierung von 5 ppm zu erzielen, wohingegen Tranilast (Substanz **15,** Tabelle 1a, nicht erfindungsgemäß)unter identischen Versuchsbedingungen selbst bei der höchsten Dosierung von 50 ppm den Histamin-Release lediglich zu 21% zu hemmen vermag.

Eine gegenüber Tranilast signifikant höhere Wirksamkeit beobachtet man auch bei Anthranilsäureamiden mit nur einer freien Hydroxyl-Funktion im Zimtsäure-/Dihydrozimtsäure-Teil, und zwar insbesondere bei deren Anordnung in Position 4 des Zimtsäure-Dihydrozimtsäure-Teils-Anteils (Tabelle 1 a: Substanzen 4-10, erfindungsgemäß sind davon die Substanzen 5 und 8). Die prozentuale Rate der Histaminfreisetzungs-Inhibition bei einer Dosierung von 50 ppm liegt hier im Bereich von 37 - 49%. Zusätzliche Substituenten im Anthranilsäure-Teil führen auch hier zu keiner signifikanten Wirksamkeitserhöhung. Auch innerhalb der Gruppe der Anthranilsäureamide mit nur einer freien Hydroxylfunktion im Zimtsäure-/Dihydrozimtsäure-Teil ist somit eine im direkten Vergleich zu Tranilast deutlich höhere Wirksamkeit zu verzeichnen.

Eine gegenüber Tranilast höhere Wirksamkeit beobachtet man auch bei Anthranilsäureamiden mit nur einer freien Hydroxylfunktion im Anthranilsäurerest, und zwar insbesondere bei deren Anordnung in Position 4 des Anthranilsäure-Restes (vgl. Tabelle 1: Substanzen 11-13, erfindungsgemäß ist davon Substanz 11). Die prozentuale Rate der Histaminfreisetzungs-Inhibition bei einer Dosierung von 50 ppm liegt hier im Bereich von 30-36 %.

Die Substanzen **2-13** sind exemplarische Vertreter einer bevorzugten Untergruppe von Verbindungen der Formel 1 (erfindungsgemäß sind davon die Substanzen 5, 8 und 11), für die gilt:
n = 1 und
zudem gilt:
   m = 1, 2 oder 3,
mit der Maßgabe, dass X zumindest einmal aus der Gruppe gewählt ist, die aus OH oder OAcyl besteht
und/oder
p = 1 oder 2,
mit der Maßgabe, dass Y zumindest einmal aus der Gruppe gewählt ist, die aus OH und OAcyl besteht.

Einen im Vergleich mit den vorbezeichneten Substanzen nicht mehr zufriedenstellenden inhibierenden Effekt hinsichtlich der Histaminfreisetzung üben die Substanzen 14-19 aus, zu denen auch Tranilast (Substanz 15) gehört.

Die vergleichsweise niedrige Wirksamkeit des Tranilast stimmt überein mit den Untersuchungen von Hachisuka et al. (Arch Dermatol Res Vol.280, p.158-162; 1988). Die Autoren konnten eine 50%-ige Inhibition der Histamin-Freisetzung erst bei einer Konzentration von 327 ppm feststellen. Augenscheinlich geht die Alkylierung der vicinalen Hydroxyl-Funktionen im Zimtsäure- oder Dihydrozimtsäure-Teil mit einer signifikanten Wirksamkeitsverringerung einher.

Entsprechende Struktur-Aktivitätsbetrachtungen innerhalb der Gruppe der Verbindungen mit Benzoesäure-Partialstruktur (Dianthramide) führten zu vergleichbaren Ergebnissen. Hier besitzen die Verbindungen mit zwei vicinalen Hydroxyl-Funktionen in den Positionen 3 und 4 des Benzoesäure-Rests (vgl. Tabelle 1b; vgl. insbesondere Substanz **20**) die stärkste Wirksamkeit. Dianthramide mit mindestens einer freien Hydroxyl-Funktion im Benzoesäure-Teil des Moleküls zeigen ebenfalls eine zwar geringfügig schwächere aber dennoch gute Wirksamkeit.

Die in Tabelle 1 b aufgelisteten Verbindungen 20-29 sind exemplarische Vertreter einer bevorzugten Untergruppe von Verbindungen der Formel 1, für die gilt: n = 0.

### 4.2 Anthranilsäureamid-Fraktionen pflanzlichen Ursprungs

Neben synthetisch hergestellten Avenanthramiden und Dianthramiden wurden auch verschiedene Pflanzenextrakte im Hinblick auf deren Eignung zur Inhibition der Substanz-P induzierten Freisetzung von Histamin aus Mastzellen untersucht. Tabelle 2 zeigt beispielhaft die Ergebnisse des direkten Vergleichs eines Haferextraktes (vgl. Tabelle 2: Probe GS10100-A, Haferextrakt standardisiert auf 500 ppm Σ Avenanthramide 3, 4 und 5; Formeln: vgl. auch Tabelle 1 a;) und eines synthetischen Rekonstituts enthaltend insgesamt 500 ppm Σ synthetischer Avenanthramide 3, 4 (nicht erfindungsgemäß) und 5 (erfindungsgemäß) (Probe GS10100-B, vgl. auch Tabelle 1a,) in exakt vergleichbaren Mengenverhältnissen.

Der direkte Vergleich beider Proben beweist, dass die Inhibition der Substanz-P induzierten Freisetzung von Histamin aus Mastzellen im wesentlichen durch die im Haferextrakt enthaltenen Avenanthramide 3, 4 und 5 (vgl. Tabelle 1a) hervorgerufen wird.

Dieser Befund zeigt generell, dass neben erfindungsgemäßen synthetischen Verbindungen auch Pflanzenextrakte und daraus hergestellte Fraktionen, die diese Substanzen in standardisierter Menge enthalten vorzüglich als Inhibitoren der Substanz P induzierten Freisetzung von Histamin aus Mastzellen einsetzbar sind und somit Entzündungen, Rötungs- und Juckreizreaktionen vorbeugen können und/oder zu deren Linderung beitragen können.

### 5. Übersicht über ausgewählte bereits bekannte und bislang unbekannte Substanzen zur Verwendung als Histaminfreisetzungs-Inhibitoren:

In den Tabellen 3 und 4 sind exemplarisch zum einen bislang unbekannte und zum anderen bereits bekannte Substanzen aufgelistet, die vorzugsweise als Histaminfreisetzungs-Inhibitoren eingesetzt werden. Die Substanzen 2-29 finden sich bereits in den Tabellen 1 a und 1 b, die Substanzen 30-80 finden sich dort nicht.

Die in den Tabellen 3 und 4 enthaltenen Substanzen sind allesamt Säuren, es sei jedoch darauf hingewiesen, dass auch die korrespondierenen Ester (mit R³= Alkyl statt R³= H) und die korrespondierenden pharmazeutisch akzeptablen Salze in gleicher Weise eingesetzt werden können.

### 6. Zusammenfassung der Untersuchungsergebnisse und ergänzende Bemerkungen:

Die durchgeführten Untersuchungen zeigten überraschenderweise, dass die erfindungsgemäßen Verbindungen der Formel 1 in gegenüber Tranilast wesentlich niedrigeren Einsatzkonzentrationen die Substanz P induzierte Freisetzung von Histamin aus Mastzellen inhibieren und somit aufgrund der niedrigeren therapeutischen Einsatzkonzentration und dem damit verbundenen geringeren toxikologischen Risikopotential bevorzugt als Wirkstoffe einsetzbar sind. Bei den erfindungsgemäß einsetzbaren Verbindungen bzw. Mischungen kann es sich um hochreine Syntheseprodukte, um reine Isolate aus Pflanzenextrakten wie z.B. aus Hafer (*Avena sativa*) oder Nelkenarten *(Dianthus* spec.) oder um spezifische Extrakt-Fraktionen aus Pflanzen wie Hafer (*Avena sativa*) oder Nelken (*Dianthus* sp.) handeln, die die bevorzugt einzusetzenden erfindungsgemäßen Verbindungen in hoher Konzentration enthalten.

Anstelle der untersuchten Verbindungen können vorzugsweise auch Verbindungen (Prekursoren), deren Hydroxyl-Funktionen im Zimtsäure-/Dihydrozimtsäure-oder im Benzoesäure-Teil acyliert sind, eingesetzt werden (X oder Y = OAcyl wobei Acyl = CO-R mit R = -CH₃, linearer oder verzweigter Alkylrest mit 2-30 C-Atomen). Darüber hinaus können vorzugsweise auch Prekursoren eingesetzt weren, deren Carboxylfunktionen im Anthranilsäure-Teil alkyliert sind (mit R³ = -CH₃, lineare oder verzweigte Alkylreste der Kettenlängen C2 bis 30). Entsprechend acylierte oder alkylierte Verbindungen der Formel 1 penetrieren bei topischer Applikation sehr gut in tiefere Hautschichten. Dort werden sie durch die in humaner und tierischer Haut endogen vorhandenen unspezifischen Esterasen gespalten, sodass das eigentliche Wirkprinzip erst am Wirkort freigesetzt wird.

Die Einsatzkonzentration der erfindungsgemäß einsetzbaren Verbindungen der Formel 1 liegt je nach Substanz im Konzentrationsbereich von 0,0001 bis 10 Gew.-% und bevorzugt im Konzentrationsbereich von 0,001 - 1 Gew.-%, bezogen auf die Gesamtmasse eines anwendungsbereiten kosmetischen oder pharmazeutischen Endprodukts.

Die erfindungsgemäß verwendeten Histaminfreisetzungs-Inhibitoren lassen sich ohne Schwierigkeiten in gängige kosmetische oder dermatologische/keratologische Formulierungen wie u.a. Pumpsprays, Aerosolsprays, Cremes, Shampoos, Salben, Tinkturen, Lotionen, Nagelpflegeprodukte (z.B. Nagellacke, Nagellackentferner, Nagelbalsame) und dergleichen einarbeiten. Hierbei ist es auch möglich und in manchen Fällen vorteilhaft, die erfindungsgemäßen Histaminfreisetzungs-Inhibitoren mit weiteren Wirkstoffen zu kombinieren, beispielsweise mit anderen evtl. sogar synergistisch verstärkenden Histaminfreisetzungs-Inhibitoren oder mit entzündungs-hemmenden und/oder juckreiz- und rötungsreduzierenden Substanzen, deren Wirkung auf einem anderen Wirkprinzip wie zum Beispiel der Inhibition der Freisetzung von Entzündungsmediatoren (unter anderem Leukotrienen, Prostaglandinen, oder Cytokinen) beruht. Die erfindungsgemäßen Histaminfreisetzungs-Inhibitoren enthaltenden kosmetischen und/oder dermatologischen/keratologischen Formulierungen können hierbei ansonsten wie üblich zusammengesetzt sein und zur Behandlung der Haut und/oder der Haare im Sinne einer dermatologischen/keratologischen Behandlung oder einer Behandlung im Sinne der pflegenden Kosmetik dienen. Sie können aber auch in Schminkprodukten in der dekorativen Kosmetik eingesetzt werden.

Kosmetische Zubereitungen, die erfindungsgemäße Histaminfreisetzungs-Inhibitoren enthalten, können auch weitere entzündungshemmende bzw. rötungs- und juckreiz-lindernde Wirkstoffe enthalten. Es können hierbei alle für kosmetische und/oder dermatologische Anwendungen geeigneten oder gebräuchlichen entzündungshemmenden bzw. rötungs- und juckreizlindernden Wirkstoffe verwendet werden. Vorteilhaft werden als entzündungshemmende bzw. rötungs- und juckreizlindernde Wirkstoffe steroidale entzündungshemmende Substanzen vom Kortikosteroiden-Typ. wie z.B. Hydrocortison, Dexamethason, Dexamethasonphosphat, Methylprednisolon oder Cortison, wobei die Auflistung durch Zusatz weiterer steroidaler Entzündungshemmer erweiterbar ist. Auch nichtsteroidale Entzündungshemmer können eingesetzt werden. Beispielhaft erwähnt werden sollen hier Oxicame wie Piroxicam oder Tenoxicam; Salicylate wie Aspirin, Disalcid, Solprin oder Fendosal; Essigsäre-Derivate wie Diclofenac, Fenclofenac, Indomethacin, Sulindac, Tolmetin, oder Clindanac; Fenamate wie Mefenamic, Meclofenamic, Flufenamic oder Niflumic; Propionsäure-Derivate wie Ibuprofen, Naproxen, Benoxaprofen oder Pyrazole wie Phenylbutazon, Oxyphenylbutazon, Febrazon oder Azapropazon. Alternativ können natürliche entzündungshemmende bzw. rötungs- und juckreizlindernde Stoffe eingesetzt werden. Einsetzbar sind Pflanzenextrakte, spezielle hochwirksame Pflanzenextrakt-Fraktionen sowie aus Pflanzenextrakten isolierte hochreine Wirksubstanzen. Besonders bevorzugt sind Extrakte , Fraktionen und Wirksubstanzen aus Kamille, Aloe vera, Commiphora-Arten, Rubia-Arten, Weiden, Weidenröschen, sowie Reinsubstanzen wie u.a. Bisabolol, Apigenin-7-glucosid, Boswelliasäure, Phytosterole, Glycyrrhizinsäure, Glabridin oder Licochalkon A. Die Histaminfreisetzungs-Inhibitpren enthaltenden Formulierungen können auch Gemische aus 2 oder mehreren weiteren antiinflammatorischen Wirkstoffen enthalten.

Kosmetische Zubereitungen, die erfindungsgemäße Histaminfreisetzungs-Inhibitoren enthalten, können auch Wirkstoffe zur Konservierung enthalten, wobei alle für kosmetische und/oder dermatologische Anwendungen geeigneten oder gebräuchlichen Konservierungsmittel verwendet werden können. Vorteilhaft werden Konservierungsmittel gewählt wie u.a. Benzoesäure, ihre Ester und Salze, Propionsäure und ihre Salze, Salicylsäure und ihre Salze, 2,4-Hexadiensäure (Sorbinsäure) und ihre Salze, Formaldehyd und Paraformaldehyd, 2-Hydroxybiphenylether und seine Salze, 2-Zinksulfidopyridin-N-oxid, Anorganische Sulfite und Bisulfite, Natriumjodat, Chlorbutanolum, 4-Hydroxybenzoesäure, ihre Salze und Ester, Dehydratcetsäure, Ameisensäure, 1,6-Bis(4-amidino-2-bromphenoxy)-n-hexan und seine Salze, das Natriumsalz der Ethylquecksilber-(II) thiosalicylsäure, Phenylquecksilber und seine Salze, 10-Undecylensäure und ihre Salze, 5-Amino-1,3-bis(2-ethylhexyl)-5-methyl-hexahydropyrimidin, 5-Brom-5-nitro-1,3-dioxan, 2-Brom-2-nitro-1,3-propandiol, 2,4-Dichlorbenzylalkohol, N-(4-Chlorphenyl)-N'-(3,4-dichlorphenyl)-harnstoff, 4-Chlor-m-kresol, 2,4,4'-Trichlor-2'-hydroxy-diphe-nylether, 4-Chlor-3,5-dimethylphenol, 1,1'-Methylen-bis(3-(1-hydroxymethyl-2,4-dioximidazolidin-5-yl)harnstoff), Poly-(hexamethylen-diguanid)-hydrochlorid, 2-Phenoxyethanol, Hexamethylentetramin, 1-(3-Chloroallyl)-3,5,7-triaza-1-azonia-adamantanchlorid, 1(4-Chlorphenoxy) 1 (1 H-imidazol-1-yl)-3,3-dimethyl-2-buta-non, 1,3-Bis-(hydroxy-methyl)-5,5-dimethyl-2,4-imidazolidindion, Benzylalkohol, Octopirox, 1,2-Dibrom-2,4-dicyanobutan, 2,2'-Methylen-bis(6-brom-4-chlor-phenol), Bromchlorophen, Mischung von 5-Chlor-2-methyl-3(2H)-isothiazolinon und 2-Methyl-3(2H)isothiazlinon mit Magnesiumchlorid und Magnesiumnitrat, 2-Benzyl-4-chlorphenol, 2-Chloracetamid, Chlorhexidin, Chlorhexidinacetat, Chlorhexidingluconat, Chlorhexidinhydrochlorid, 1-Phenoxy-propan-2-ol, N-Alkyl(C₁₂-C₂₂)trimethyl-ammoniumbromid und -chlorid, 4,4-Dimethyl-1,3-oxazolidin, N-Hydroxymethyl-N-(1,3-di(hydroxymethyl)-2,5-dioxoimida-zolidin-4-yl)-N'-hydroxy-methylharnstoff, 1,6-Bis(4-amidino-phenoxy)-n-hexan und seine Salze, Glutaraldehyd, 5-Ethyl-1-aza-3,7-dioxabicyclo(3.3.0)octan, 3-(4-Chlorphenoxy)-1,2-propandiol, Hyamine, Alkyl-(C₈-C₁₈)-dimethy)-benzyl-ammoniumchlorid, Alkyl-(C₈-C₁₈)-dimethyl-benzylammoniumbromid, Alkyl-(C₈-C₁₈)-dimethyl-benzyl-ammonium-saccharinat, Benzylhemiformal, 3-Jod-2-propinyl-butylcarbamat, Natrium-hydroxymethyl-aminoacetat oder Natrium-hydroxymethyl-aminoacetat.

In den kosmetischen Zubereitungen, die erfindungsgemäße Histaminfreisetzungs-Inhibitoren enthalten, können auch weitere antibakterielle oder antimykotische Wirkstoffe besonders vorteilhaft eingesetzt werden, wobei alle für kosmetische und/oder dermatologische Anwendungen geeigneten oder gebräuchlichen antibakteriellen oder antimykotischen verwendet werden können. Vorteilhaft sind hier neben der großen Gruppe der klassischen Antibiotika insbesondere die für Kosmetik relevante Produkte wie Triclosan, Climbazol, Octoxyglycerin, Octopirox (1-Hydroxy-4-methyl-6-(2,4,4-trimethylpentyl)-2(1 1H)-pyridone, 2-aminoetha-nol), Chitosan, Farnesol, Glycerinmonolaurat oder Kombinationen der genannten Substanzen, die u.a. gegen Achselgeruch, Fußgeruch oder Schuppenbildung eingesetzt werden.

Darüberhinaus können die synergistischen Mischungen von erfindungsgemäßen Histaminfreisetzungs-Inhibitoren auch in Kombination mit schweißhemmenden Wirkstoffen (Antitranspirantien) besonders vorteilhaft zur Bekämpfung von Körpergeruch eingesetzt werden. Als schweißhemmende Wirkstoffe kommen vor allem Aluminiumsalze wie Aluminiumchlorid, Aluminiumchlorhydrat, -nitrat, -sulfat, -acetat usw. zum Einsatz. Daneben kann aber auch die Verwendung von Zink-, Magnesium- und Zirkoniumverbindungen vorteilhaft sein. Für die Anwendung in kosmetischen und dermatologischen Antitranspirantien haben sich im wesentlichen die Aluminiumsalze und - in etwas geringerem Maße - Aluminium/Zirkoniumsalz-Kombinationen durchgesetzt. Daneben erwähnenswert sind die teilneutralisierten und damit besser hautverträglichen, aber nicht ganz so wirksamen Aluminiumhydroxychloride. Neben Aluminiumsalzen kommen auch weitere Substanzen in Betracht wie zum Beispiel a) eiweißfällende Substanzen wie u.a. Formaldehyd, Glutaraldehyd, natürliche und synthetische Gerbstoffe sowie Trichloressigsäure, die einen oberflächlichen Verschluss der Schweißdrüsen herbeiführen b) Lokalanästhetika (u.a. verdünnte Lösungen von z.B. Lidokain, Prilokain oder Gemischen derartiger Substanzen), die durch Blockade der peripheren Nervenbahnen die sympathische Versorgung der Schweißdrüsen ausschalten, c) Zeolithe vom X-, A- oder Y-Typ, die neben der Reduktion der Schweißsekretion auch als Adsorbentien für schlechte Gerüche fungieren und d) Botulinustoxin (Toxin des Bakteriums *Chlostridium botulinum),* welches auch bei Hyperhidrose, einer krankhaft erhöhten Schweißsekretion, zum Einsatz gelangt und dessen Wirkung auf einer irreversiblen Blockierung der Freisetzung der für die Schweißsekretion relevanten Transmittersubstanz Acetylcholin beruht.

In den kosmetischen Zubereitungen, die erfindungsgemäße Histaminfreisetzungs-Inhibitoren enthalten, kann auch eine Kombination mit (Metall)-Chelatoren vorteilhaft sein, wobei alle für kosmetische und/oder dermatologische Anwendungen geeigneten oder gebräuchlichen Metallchelatoren verwendet werden können. Bevorzugt einzusetzende (Metall)-Chelatoren sind u.a α-Hydroxyfettsäuren, Phytinsäure, Lactoferrin, α-Hyroxysäuren wie u.a. Zitronensäure, Milchsäure und Äpfelsäure sowie Huminsäuren, Gallensäuren, Gallenextrakte, Bilirubin, Biliverdin bzw. EDTA, EGTA und deren Derivate.

Zur Anwendung werden die erfindungsgemäßen, Histaminfreisetzungs-Inhibitoren enthaltenden Formulierungen in der für Kosmetika und Dermatika üblichen Weise auf die Haut und/oder die Haare in ausreichender Menge aufgebracht. Besondere Vorteile bieten dabei kosmetische und dermatologische Zubereitungen, die eine erfindungsgemäße Mischung enthalten und zusätzlich als Sonnenschutzmittel wirken. Vorteilhaft enthalten diese Zubereitungen mindestens einen UVA-Filter und/oder mindestens einen UVB-Filter und/oder mindestens ein anorganisches Pigment. Die Zubereitungen können dabei in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Wie erwähnt können Zubereitungen, die erfindungsgemäße Histaminfreisetzungs-Inhibitoren enthalten, vorteilhaft mit Substanzen kombiniert werden, die UV-Strahlung im UVB-Bereich absorbieren, wobei die Gesamtmenge der Filtersubstanzen z.B. 0,01 Gew.-% bis 40 Gew.-%, vorzugsweise 0,1% bis 10 Gew.-%, insbesondere 1,0 bis 5,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen. Enthalten die erfindungsgemäßen Zubereitungen UVB-Filtersubstanzen, können diese öllöslich oder wasserlöslich sein. Vorteilhafte öllösliche UVB-Filter sind z.B.: 3-Benzylidencampher-Derivate, vorzugsweise 3-(4-Methylbenzyli-den)campher, 3-Benzylidencampher; 4-Aminobenzoesäure-Derivate, vorzugs-weise 4-(Dimethylamino)-benzoesäure(2-ethylhexyl) ester, 4-(Dimethyl-amino) benzoesäureamylester; Ester der Zimtsäure, vorzugsweise 4-Methoxyzimtsäure(2-ethylhexyl)ester, 4-Methoxyzimtsäureisopentylester; Ester der Salicylsäure, vorzugsweise Salicylsäure(2-ethylhexyl)ester, Salicylsäure (4isopropylbenzyl) ester, Salicylsäurehomomenthylester, Derivate des Benzophenons, vorzugsweise 2-Hydroxy-4-methoxybenzophenon,2-Hydroxy-4-methoxy-4'-methylbenzo- phenon, 2,2'-Dihydroxy-4-methoxybenzophenon; Ester der Benzalmalonsäure, vorzugsweise 4-Methoxybenzalmalonsäuredi(2-ethylhexyl) ester, 2,4,6-Trianilino-(p-carbo-2'-ethyl-1'-hexyloxy)-1,3,5-triazin. Vorteilhafte wasserlösliche UVB-Filter sind z.B. Salze der 2-Phenylbenzimidazol-5-sulfonsäure wie ihr Natrium-, Kalium- oder ihr Triethanolammonium-Salz, sowie die Sulfonsäure selbst; Sulfonsäure-Derivate von Benzophenonen, vorzugsweise 2-Hydroxy-4-methoxybenzophenon-5-sulfonsäure und ihre Salze; Sulfonsäure-Derivate des 3-Benzylidencamphers, wie z.B. 4-(2-Oxo-3-bornylidenmethyl)benzolsulfonsäure, 2-Methyl-5-(2-oxo-3-bornyliden-methyl)sulfonsäure und ihre Salze sowie das 1,4-di(2-oxo-10-Sulfo-3-bornylidenmethyl)benzol und dessen Salze (die entsprechenden 10-Sulfato-verbindungen, beispielsweise das entsprechende Natrium-, Kalium- oder Triethanolammonium-Salz), auch als Benzol-1,4-di(2-oxo-3-bomylidenmethyl-10-Sulfonsäure bezeichnet.

Die vorstehende Liste der genannten UVB-Filter, die in Kombination mit den erfindungsgemäßen Histaminfreisetzungs-Inhibitoren verwendet werden können, soll selbstverständlich nicht als abschließend verstanden werden. Es kann auch von Vorteil sein, UVA-Filter einzusetzen, wie sie üblicherweise in kosmetischen Zubereitungen enthalten sind. Bei diesen Substanzen handelt es sich vorzugsweise um Derivate des Dibenzoylmethans, insbesondere um 1-(4'-tert.-Butylphenyl)-3-(4'-methoxyphenyl) propan-1,3-dion und um 1-Phenyl-3-(4'-isopropylphenyl)propan-1,3-dion. Es können die für die UVB-Kombination verwendeten Mengen eingesetzt werden.

Die erfindungsgemäßen Histaminfreisetzungs-Inhibitoren können in kosmetischen Zubereitungen vorteilhaft auch mit weiteren kosmetischen Hilfsstoffen kombiniert werden, wie sie üblicherweise in solchen Zubereitungen verwendet werden, z.B. Antioxidantien, Parfümöle, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate. Erfindungsgemäß können hierbei alle denkbaren für kosmetische und/oder dermatologische Anwendungen geeigneten oder gebräuchlichen Antioxidantien, Parfümöle, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse, Alkohole, Polyole, Polymere, Schaumstabilisatoren, Elektrolyte, organische Lösungsmittel oder Silikonderivate verwendet werden.

In Histaminfreisetzungs-Inhibitoren enthaltenden Formulierungen zur topischen prophylaktischen oder kosmetischen Behandlung der Haut ist regelmäßig ein hoher Anteil an pflegenden Substanzen vorteilhaft sein. Gemäß einer bevorzugten Ausführungsform enthalten die Zusammensetzungen ein oder mehrere pflegende tierischen und/oder pflanzliche Fetten und Ölen wie Olivenöl, Sonnenblumenöl, raffiniertem Sojaöl, Palmöl, Sesamöl, Rapsöl, Mandelöl, Borretschöl, Nachtkerzenöl, Kokosöl, Sheabutter, Jojobaöl, Spermöl, Rindertalg, Klauenöl und Schweineschmalz sowie gegebenfalls weitere pflegende Bestandteile wie zum Beispiel Fettalkohole mit 8-30 C-Atomen. Die verwendeten Fettalkohole können hierbei gesättigt oder ungesättigt bzw. linear oder verzweigt sein. Einsetzbar sind zum Beispiel Decanol, Decenol, Octanol, Octenol, Dodecanol, Dodecenol, Octadienol, Decadienol, Dodecadienol, Oleylalkohol, Ricinolalkohol, Erucaalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung durch weitere strukturchemisch verwandte Alkohole beliebig erweiterbar wäre. Die Fettalkohole stammen bevorzugt von natürlichen Fettsäuren ab, wobei sie üblicherweise aus den korrespondierenden Estern der Fettsäuren durch Reduktion hergestellt werden. Einsetzbar sind weiterhin Fettalkoholfraktionen, die durch Reduktion aus natürlich vorkommenden Fetten und fetten Ölen, wie z.B. Rindertalg, Erdnussöl, Rüböl, Baumwollsamenöl, Sojaöl, Sonnenblumenöl, Palmkernöl, Leinöl, Maisöl, Rizinusöl, Rapsöl, Sesamöl, Kakaobutter und Kokosfett entstehen.

Zu pflegenden Substanzen, die sich vorzüglich mit den erfindungsgemäßen Histaminfreisetzungs-Inhibitoren kombinieren lassen, zählen darüberhinaus auch
- Ceramide, wobei man unter Ceramiden N-Acylsphingosine (Fettsäreamide des Sphingosins) oder synthetische Analoga solcher Lipide (sogenannte Pseudo-Ceramide) versteht, die das Wasserhaltevermögen des Stratum Corneums deutlich verbessern.
- Phospholipide, beispielsweise Sojalecithin, Ei-Lecithin und Kephaline
- Vaseline, Paraffin- und Silikonöle; zu letzteren zählen unter anderem Dialkyl- und Alkylarylsiloxane wie Dimethylpolysiloxan und Methylphenylpolysiloxan, sowie deren alkoxylierte und quaternierte Derivate

Den erfindungsgemäßen, Histaminfreisetzungs-Inhibitoren enthaltenden Formulierungen können vorteilhaft auch tierische und/oder pflanzliche Proteinhydrolysate zugesetzt werden. Vorteilhaft sind insoweit insbesondere Elastin-, Kollagen-, Keratin-, Milcheiweiß-, Sojaprotein-, Haferprotein-, Erbsenprotein-, Mandelprotein-, und Weizenproteinfraktionen oder entsprechende Proteinhydrolysate aber auch deren Kondensationsprodukte mit Fettsäuren sowie quarternisierte Proteinhydrolysate, wobei die Verwendung pflanzlicher Proteinhydrolysate bevozugt ist.

Sofern eine kosmetische oder dermatologische, erfindungsgemäße Histaminfreisetzungs-Inhibitoren enthaltende Zubereitung eine Lösung oder Lotion darstellt, können als Lösungsmittel verwendet werden:
- Wasser oder wässrige Lösungen;
- fette Öle, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Kosmetische Zubereitungen, die erfindungsgemäße Histaminfreisetzungs-Inhibitoren enthalten, können auch Antioxidantien enthalten, wobei alle für kosmetische und/oder dermatologische Anwendungen geeigneten oder gebräuchlichen Antioxidantien verwendet werden können. Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L- - Carnosin, D-Carnosin, L-Carnosin und deren Derivate (z.B. Anserin), Carotinoide, Carotine (z.B. α-Carotin, ß-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathionin-sulfoximin) in sehr geringen verträglichen Dosierungen, ferner (Metall)-Chelatoren, z.B. α-Hydroxyfettsäuren, Palmitinsäure, Phytinsäure, Lactoferrin, α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Coniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO4) Selen und dessen Derivate (z.B. Selenmethionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Kosmetische Zubereitungen, die erfindungsgemäße Histaminfreisetzungs-Inhibitoren enthalten, können auch Vitamine und Vitaminvorstufen enthalten, wobei alle für kosmetische und/oder dermatologische Anwendungen geeigneten oder gebräuchlichen Vitamine oder Vitaminvorstufen verwendet werden können. Erwähnenswert sind hier insbesondere Vitamine und Vitaminvorstufen wie Tocopherole, Vitamin A, Niacinsäure und Niacinsäureamid, weitere Vitamine des B-Komplexes, insbesondere Biotin und Vitamin C. Weiterhin innnerhalb dieser Gruppe bevorzugt verwendet werden Panthenol und dessen Derivate, insbesondere die Ester und Ether des Panthenols sowie kationisch derivatisierte Panthenole wie z.B. Panthenoltriacetat, Panthenolmonoethylether und dessen Monoacetat sowie kationische Panthenolderivate.

Kosmetische Zubereitungen, die Histaminfreisetzungs-Inhibitoren enthalten, können auch Wirkstoffe mit hautaufhellender Wirkung enthalten. Erfindungsgemäß können hierbei alle für kosmetische und/oder dermatologische Anwendungen geeigneten oder gebräuchlichen hautaufhellenden Wirkstoffe verwendet werden. Vorteilhafte hautaufhellende Wirkstoffe sind insoweit Kojicacid, Hydrochinon, Arbutin, Ascorbinsäure, Magnesiumascorbylphosphat, Süßholzwurzel-Extrakten sowie deren Bestandteile Glabridin oder Licochalkon A, oder Extrakte von Rumex- und Ramulus-Arten, Extrakte aus Kieferarten (Pinus) oder Extrakte aus Vitis-Arten die u.a. hautaufhellende Stilbenderivate enthalten.

Kosmetische Zubereitungen, die erfindungsgemäße, Histaminfreisetzungs-Inhibitoren enthalten, können auch Wirkstoffe mit hautbräunender Wirkung enthalten. Es können insoweit alle für kosmetische und/oder dermatologische Anwendungen geeigneten oder gebräuchlichen hautbräunenden Wirkstoffe verwendet werden. Beispielhaft sei hier das Dihydroxyaceton (DHA; 1,3-Dihydroxy-2-propanon) erwähnt. DHA kann sowohl in monomerer als auch in dimerer Form vorliegen, wobei in kristalliner Form der Anteil an Dimeren überwiegt.

Kosmetische Zubereitungen, die erfindungsgemäße Histaminfreisetzungs-Inhibitoren enthalten, können auch Mono- Di- und Oligosaccharide wie beispielsweise Glucose, Galactose, Fructose, Mannose, Fruchtzucker und Lactose enthalten.

Kosmetische Zubereitungen, die erfindungsgemäße Histaminfreisetzungs-Inhibitoren enthalten, können auch Pflanzenextrakte enthalten, die üblicherweise durch Extraktion der gesamten Pflanze, in einzelnen Fällen aber auch ausschließlich aus Blüten und/oder Blättern, Holz, Rinde oder Wurzeln der Pflanze hergestellt werden. Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflegemittel und Waschmittel e.V. (IKW), Frankfurt beginnenden Tabelle aufgeführt sind. Vorteilhaft sind insbesondere die Extrakte aus Aloe, Hamamelis, Algen, Eichenrinde, Weidenröschen, Brennnessel, Taubnessel, Hopfen, Kamille, Schafgarbe, Arnika, Calendula, Klettenwurzel, Schachtelhalm, Weißdorn, Lindenblüten, Mandel, Fichtennadel, Rosskastanie, Sandelholz, Wacholder, Kokosnuss, Mango, Aprikose, Orange, Zitrone, Limette, Grapefruit, Apfel, grünem Tee, Grapefruitsamen, Weizen, Hafer, Gerste, Salbei, Thymian, Quendel, Rosmarin, Birke, Malve, Wiesenschaumkraut, Weidenrinde, Hauhechel, Huflattich, Eibisch, Ginseng und Ingwerwurzel. Besonders bevorzugt sind dabei die Extrakte aus Aloe vera, Kamille, Algen, Rosmarin, Calendula, Ginseng, Gurke, Salbei, Brennnessel, Lindenblüten, Arnika, und Hamamelis. Es können auch Mischungen aus zwei oder mehreren, Pflanzenextrakten eingesetzt werden. Als Extraktionsmittel zur Herstellung der genannten Pflanzenextrakte können u.a. Wasser, Alkohole sowie deren Mischungen verwendet werden. Unter den Alkoholen sind dabei niedere Alkohole wie Ethanol und Isopropanol, aber auch mehrwertige Alkohole wie Ethylenglykol, Propylenglykol und Butylenglykol und zwar sowohl als alleiniges Extraktionsmitttel als auch in Mischungen mit Wasser bevorzugt. Die Pflanzenextrakte können erfindungsgemäß sowohl in reiner als auch in verdünnter Form eingesetzt werden.

Kosmetische Zubereitungen, die efindungsgemäße, Histaminfreisetzungs-Inhibitoren enthalten, können zumal wenn kristalline oder mikrokristalline Festkörper, beispielsweise anorganische Mikropigmente in die erfindungsgemäßen Zubereitungen eingearbeitet werden sollen, auch anionische, kationische, nichtionische und/oder amphotere Tenside enthalten. Tenside sind amphiphile Stoffe, die organische, unpolare Substanzen in Wasser lösen können. Bei den hydrophilen Anteilen eines Tensidmoleküls handelt es sich dabei meist um polare funktionelle Gruppen, beispielweise -COO⁻, - OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch. Typisch für nicht-ionische Tenside sind Polyether-Ketten. Nicht-ionische Tenside bilden in wässrigem Medium keine Ionen.

### A. Anionische Tenside

Vorteilhaft zu verwendende anionische Tenside sind Acylaminosäuren (und deren Salze), wie
- Acylglutamate, beispielsweise Natriumacylglutamat, Di-TEA-palmitoylaspartat und Natrium Capryl/ Capringlutamat,
- Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
- Sarcosinate, beispielsweise Myristoyl Sarcosin, TEA-lauroylsarcosinat, Natriumlauroylsarcosinat und Natriumcocoylsarkosinat,
- Taurate, beispielsweise Natriumlauroyltaurat und Natriummethylcocoyltaurat,
- Acyllactylate, Lauroyllactylat, Caproyllactylat
- Alaninate

Carbonsäuren und Derivate, wie
- beispielsweise Laurinsäure, Aluminiumstearat, Magnesiumalkanolat und Zinkundecylenat,
- Ester-Carbonsäuren, beispielsweise Calciumstearoyllactylat, Laureth-6 Citrat und Natrium PEG-4 Lauramidcarboxylat,
- Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,
Sulfonsäuren und Salze, wie
- Acyl-isothionate, z.B. Natrium-/ Ammoniumcocoyl-isethionat,
- Alkylarylsulfonate,
- Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
- Sulfosuccinate, beispielsweise Dioctylnatriumsulfosuccinat, Dinatriumlaureth-sulfosuccinat, Dinatriumlaurylsulfosuccinat und Dinatriumundecylenamido MEA-Sulfosuccinat
sowie
Schwefelsäureester, wie
- Alkylethersulfat, beispielsweise Natrium-, Ammonium-, Magnesium-, MIPA-, TIPA-Laurethsulfat, Natriummyrethsulfat und Natrium C12-13 Parethsulfat,
- Alkylsulfate, beispielsweise Natrium-, Ammonium- und TEA-Laurylsulfat.

### B. Kationische Tenside

Vorteilhaft zu verwendende kationische Tenside sind
- Alkylamine,
- Alkylimidazole,
- Ethoxylierte Amine und
- Quaternäre Tenside.
   RNH₂CH₂CH₂COO⁻ (bei pH=7)
   RNHCH₂CH₂COO- B⁺ (bei pH=12) B⁺ = beliebiges Kation, z.B. Na⁺
- Esterquats

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkylamidopropylhydroxysulfain. Die verwendeten kationischen Tenside können ferner bevorzugt gewählt werden aus der Gruppe der quaternären Ammoniumverbindungen, insbesondere Benzyltrialkyl-ammoniumchloride oder -bromide, wie beispielsweise Benzyldimethylstearyl-ammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethyl-aminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
- Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
- N-Alkylaminosäuren, beispielsweise Aminopropylalkylglutamid, Alkylaminopropionsäure, Natriumalkylimidodipropionat und Lauroampho-carboxyglycinat.

### D. Nicht-ionische Tenside

Vorteilhaft zu verwendende nicht-ionische Tenside sind
- Alkohole,
- Alkanolamide, wie Cocamide MEA/ DEA/ MIPA,
- Aminoxide, wie Cocoamidopropylaminoxid,
- Ester, die durch Veresterung von Carbonsäuren mit Ethylenoxid, Glycerin, Sorbitan oder anderen Alkoholen entstehen,
- Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Poly-siloxane, propoxylierte POE-Ether und Alkylpolyglycoside wie Lauryl-glucosid, Decylglycosid undcocoglycosid.
- Sucroseester, -Ether
- Polyglycerinester, Diglycerinester, Monoglycerinester
- Methylglucosester, Ester von Hydroxysäuren

Vorteilhaft ist ferner die Verwendung einer Kombination von anionischen und/ oder amphoteren Tensiden mit einem oder mehreren nicht-ionischen Tensiden. Die oberflächenaktive Substanz kann in einer Konzentration zwischen 1 und 98 Gew.-% in den erfindungsgemäßen, Histaminfreisetzungs-Inhibitoren enthaltenden Formulierungen vorliegen, bezogen auf das Gesamtgewicht der Zubereitungen.

Kosmetische oder dermatologische Zubereitungen, die erfindungsgemäße Histaminfreisetzungs-Inhibitoren enthalten, können auch als Emulsionen vorliegen.

Die Ölphase kann vorteilhaft gewählt werden aus folgender Substanzgruppe:
- Mineralöle, Mineralwachse
- fette Öle, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Vorteilhaft einsetzbar sind Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Bevorzugte Esteröle sind Isopropylmyristat, Isopropylpalmitat, Isopropylstearat, Isopropyloleat, n-Butylstearat, n-Hexyllaurat, n-Decyloleat, Isooctylstearat, Isononylstearat, Iso-nonylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr. Auch beliebige Abmischungen solcher Öl- und Wachskomponenten sind vorteilhaft einsetzbar. In manchen Fällen ist auch vorteilhaft, Wachse, beispielsweise Cetylpalmitat, als alleinige Lipidkomponente der Ölphase einzusetzen, vorteilhaft wird die Ölphase gewählt aus der Gruppe die besteht aus 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether. Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat. Ach die Kohlenwasserstoffe Paraffinöl, Squalan und Squalen lassen sich vorteilhaft verwenden. Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden. Cyclomethicon (z.B. Decamethylcyclopentasiloxan) kann vorteilhaft als Silikonöl eingesetzt werden. Aber auch andere Silikonöle sind vorteilhaft verwendbar, beispielsweise Undecamethylcyclotrisiloxan, Polydimethylsiloxan, Poly(methyl-phenylsiloxan). Besonders vorteilhaft sind ferner Mischungen aus Cyclomethicon und Isotridecylisononanoat, aus Cyclomethicon und 2-Ethylhexylisostearat.

Die wässrige Phase von als Emulsion vorliegenden Zubereitungen, die erfindungsgemäße Histaminfreisetzungs-Inhibitoren enthalten kann umfassen: Alkohole, Diole oder Polyole niedriger C-Zahl, sowie deren Ether, vorzugsweise Ethanol, Isopropanol, Propylenglykol, Glycerin, Ethylenglykol, Ethylenglykol- monoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Als Emulsion vorliegende Zubereitungen, die erfindungsgemäße Histaminfreisetzungs-Inhibitoren enthalten, umfassen vorteilhaft einen oder mehrere Emulgatoren. O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel R-COO-(-CH₂-CH₂-O-)ₙ-H,
- der veretherten Fettsäureethoxylate der allgemeinen Formel

   R-COO-(-CH₂-CH₂-O-)ₙ-R',

   der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel
   R-COO-(-CH₂-CH₂-O-)ₙ-OOH, und n eine Zahl von 5 bis 30 darstellen,
- der Polyoxyethylensorbitolfettsäureester,
- der Alkylethersulfate der allgemeinen Formel R-O-(-CH₂-CH₂-O-)n-SO₃-H
- der Fettalkoholpropoxylate der allgemeinen Formel R-O-(-CH₂-CH(CH₃)-O-)ₙ₋H
- der Polypropylenglycolether der allgemeinen Formel

   R-O-(-CH₂-CH(CH₃)-O-)ₙ-R'
- der propoxylierten Wollwachsalkohole,
- der veretherten Fettsäurepropoxylate R-COO-(-CH₂-CH(CH₃)-O-)ₙ-R'
- der veresterten Fettsäurepropoxylate der allgemeinen Formel

   R-COO-(-CH₂-CH(CH₃)-O-)ₙ-C(O)-R'
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)-O-)ₙ-CH₂-COOH,
- der Alkylethersulfate bzw. die diesen Sulfaten zugrundeliegenden Säuren der allgemeinen Formel R-O-(-CH₂-CH(CH₃)-O-)ₙ-SO₃-H,
- der Fettalkoholethoxylate/propoxylate der allgemeinen Formel R-O-Xₙ-Yₘ-H
- der Polypropylenglycolether der allgemeinen Formel R-O-Xₙ-Yₘ-R'
- der veretherten Fettsäurepropoxylate der allgemeinen Formel R-COO-Xₙ-Yₘ-R'
- der Fettsäureethoxylate/propoxylate der allgemeinen Formel R-COO-Xₙ-Yₘ-H.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13),
Polyethylenglycol(14)stearylether (Steareth-14),
Polyethylenglycol(15)stearylether (Steareth-15),
Polyethylenglycol (16)stearylether (Steareth-16),
Polyethylenglycol(17)stearylether (Steareth-17),
Polyethylenglycol-(18)stearylether (Steareth-18),
Polyethylenglycol(19)stearylether (Steareth-19),
Polyethylenglycol(20)stearylether (Steareth-20),
Polyethylenglycol(12)isostearylether (Isosteareth-12),
Polyethylenglycol(13)isostearylether (Isosteareth-13),
Polyethylenglycol(14)isostearylether (Isosteareth-14),
Polyethylenglycol(15)isostearylether (Isosteareth-15),
Polyethylenglycol(16)isostearylether (Isosteareth-16),
Polyethylenglycol(17)isostearylether (Isosteareth-17),
Polyethylenglycol-(18)isostearylether (Isosteareth-18),
Polyethylenglycol(19)isostearylether (Isosteareth-19),
Polyethylenglycol(20)isostearylether (Isosteareth-20),
Polyethylenglycol(13)cetylether (Ceteth-13),
Polyethylenglycol(14)cetylether (Ceteth-14),
Polyethylenglycol(15)cetylether (Ceteth-15),
Polyethylenglycol(16)cetylether (Ceteth-16),
Polyethylenglycol(17)cetylether (Ceteth-17),
Polyethylenglycol(18)cetylether (Ceteth-18),
Polyethylenglycol(19)cetylether (Ceteth-19),
Polyethylenglycol(20)cetylether (Ceteth-20),
Polyethylenglycol(13)isocetylether (Isoceteth-13),
Polyethylenglycol(14)isocetylether (Isoceteth-14),
Polyethylenglycol(15)isocetylether (Isoceteth-15),
Polyethylenglycol(16)isocetylether (Isoceteth-16),
Polyethylenglycol(17)isocetylether (Isoceteth-17),
Polyethylenglycol(18)isocetylether (Isoceteth-18),
Polyethylenglycol(19)isocetylether(Isoceteth-19),
Polyethylenglycol(20)isocetylether (Isoceteth-20),
Polyethylenglycol(12)oleylether (Oleth-12),
Polyethylenglycol(13)oleylether (Oleth-13),
Polyethylenglycol(14)oleylether (Oleth-14),
Polyethylenglycol(15)oleylether (Oleth-15),
Polyethylenglycol(12)laurylether (Laureth-12),
Polyethylenglycol(12)isolaurylether (Isolaureth12).
Polyethylenglycol(13)cetylstearylether (Ceteareth-13),
Polyethylenglycol(14)cetylstearylether (Ceteareth-14),
Polyethylenglycol(15)cetylstearylether (Ceteareth-15),
Polyethylenglycol(16)cetylstearylether (Ceteareth-16),
Polyethylenglycol(17)cetylstearylether (Ceteareth-17),
Polyethylenglycol(18)cetylstearylether (Ceteareth-18),
Polyethylenglycol-(19)cetylstearylether (Ceteareth-19),

Polyethylenglycol(20)cetylstearylether (Ceteareth-20),

Es ist ferner von Vorteil, die Fettsäureethoxylate aus folgender Gruppe zu wählen:
Polyethylenglycol(20)stearat,
Polyethylenglycol(21)stearat,
Polyethylenglycol(22)stearat,
Polyethylenglycol(23)stearat,
Polyethylenglycol(24)stearat,
Polyethylenglycol(25)stearat,
Polyethylenglycol(12)isostearat,
Polyethylenglycol(13)isostearat,
Polyethylenglycol-(14)isostearat,
Polyethylenglycol(15)isostearat,
Polyethylenglycol(16)isostearat,
Polyethylenglycol(17)isostearat,
Polyethylenglycol(18)isostearat,
Polyethylenglycol(19)isostearat,
Polyethylenglycol(20)isostearat,
Polyethylenglycol(21)isostearat,
Polyethylenglycol-(22)isostearat,
Polyethylenglycol(23)isostearat,
Polyethylenglycol(24)isostearat,
Polyethylenglycol(25)isostearat,
Polyethylenglycol(12)oleat,
Polyethylenglycol(13)oleat,
Polyethylenglycol(1 4)oleat,
Polyethylenglycol(15)oleat,
Polyethylenglycol(1 6)oleat,
Polyethylenglycol(1 7)oleat,
Polyethylenglycol(1 8)oleat,
Polyethylenglycol(1 9)oleat,
Polyethylenglycol(20)oleat

Als ethoxylierte Alkylethercarbonsäure bzw. deren Salz kann vorteilhaft das Natriumlaureth-11-carboxylat verwendet werden. Als Alkylethersulfat kann Natrium Laureth 1-4 sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat,
Polyethylenglycol(21)glyceryllaurat,
Polyethylenglycol(22)glyceryllaurat,
Polyethylenglycol(23)glyceryllau rat,
Polyethylenglycol(6)glycerylcaprat/caprinat,
Polyethylenglycol(20)glyceryloleat,
Polyethylenglycol(20)glycerylisostearat,
Polyethylenglycol(18)glyceryloleat/cocoat zu wählen.

Es ist ebenfalls günstig, die Sorbitanester aus der Gruppe
Polyethylenglycol(20)sorbitanmonolaurat,
Polyethylenglycol(20)sorbitanmonostearat,
Polyethylenglycol(20)sorbitanmonoisostearat,
Polyethylenglycol(20)sorbitanmonopalmitat,
Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atomen, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether(Steareth-2), Glycerylmonolaurat, Glycerylmonocaprinat, Glycerylmonocaprylat.

Eine erfindungsgemäße Substanz der Formel 1 kann auch als Bestandteil von Duftstoffkompositionen (Riechstoffkompositionen) eingesetzt werden und aufgrund seiner spezifiischen Wirksamkeit als Inhibitor der Substanz P induzierten Freisetzung von Histamin aus Mastzellen beispielsweise einem parfümierten Fertigprodukt eine antiallergische oder juckreizlindernde Wirkung verleihen. Eine besonders bevorzugte Duftstoffkomposition umfasst (a) eine sensorisch wirksame Menge eines Duftstoffes, (b) eine z.B antiallergisch oder juckreiz-lindernd wirkende Menge einer oder mehrerer Verbindungen der Formel 1 sowie (c) ggf. einen oder mehrere Trägerstoffe und/oder Zusatzstoffe. Da der Anteil an Parfüm in einem kosmetischen Fertigprodukt häufig im Bereich von ca. 1 Gew.-% liegt, wird ein Parfüm, welches eine erfindungsgemäße Verbindung der Formel 1 enthält, vorzugsweise zu etwa 0,1-10 Gew.-% aus einer oder mehreren Verbindungen der Formel 1 bestehen. Als besonders vorteilhaft hat es sich erwiesen, dass die Substanzen der Formel 1 nur einen schwachen Eigengeruch besitzen oder gar völlig geruchlos sind; denn diese Eigenschaft prädestiniert sie insbesondere für den Einsatz als Inhibitoren der Substanz P induzierten Freisetzung von Histamin aus Mastzellen in einer Duftstoffkomposition.

Bevorzugte Ausgestaltungen und weitere Aspekte der vorliegenden Erfindung ergeben sich aus den beigefügten Patentansprüchen.

**Tabelle 1a**

| No. | Strukturformel | %-Inhibition | | |
|---|---|---|---|---|
| | | 0,5 ppm | 5 ppm | 50 ppm |
| 2 | 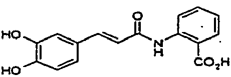 | 7 | 80 | 108 |
| 3 | 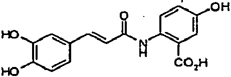 | 11 | 62 | 106 |
| 4 | 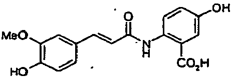 | 3 | 49 | 59 |
| 5 | 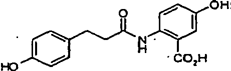 | 7 | 17 | 47 |
| 6 | 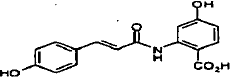 | -9 | 1 | 41 |
| 7 | 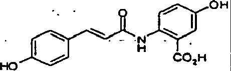 | 27 | 26 | 40 |
| 8 | 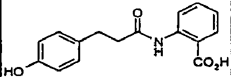 | 13 | 26 | 40 |
| 9 | 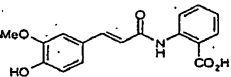 | 11 | 19 | 39 |
| 10 | 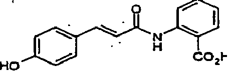 | -10 | 8 | 37 |
| 11 | 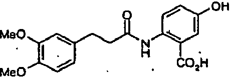 | -6 | 11 | 36 |
| 12 | 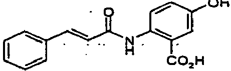 | -5 | 0 | 34 |
| 13 | 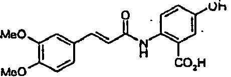 | 17 | 2 | 30 |
| 14 | 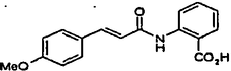 | 21 | 30 | 24 |
| 15 | 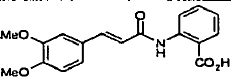 | 23 | 22 | 21 |
| 16 | 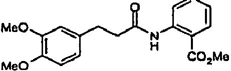 | 26 | 25 | 7 |
| 17 | 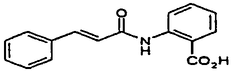 | 4 | 7 | 6 |
| 18 | 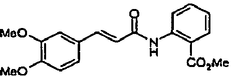 | 5 | -3 | 6 |
| 19 | 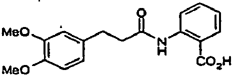 | -15 | -22 | -4 |

| Tabelle 1b: | | | | |
|---|---|---|---|---|
| | | %-Inhibition | | |
| No. | Strukturformel | 0,5 ppm | 5 ppm | 50 ppm |
| 20 | 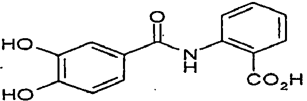 | 14 | 76 | 110 |
| 21 | 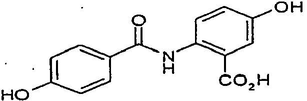 | -15 | 48 | 76 |
| 22 | 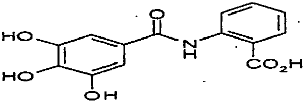 | 39 | 72 | 71 |
| 23 | 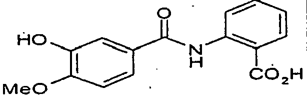 | 9 | 29 | 69 |
| 24 | 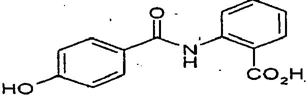 | 7 | 19 | 55 |
| 25 | 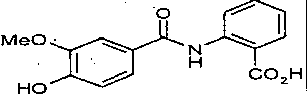 | 8 | 42 | 51 |
| 26 | 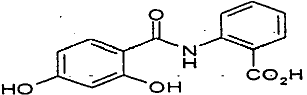 | -13 | 22 | 48 |
| 27 | 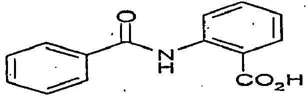 | 27 | 28 | 39 |
| 28 | 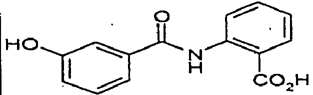 | 12 | 25 | 35 |
| 29 | 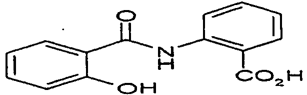 | -1 | 15 | 23 |

| Tabelle 2: | | | | |
|---|---|---|---|---|
| GS101100-A: Anthranilsäureamid-Fraktion aus Haferstrohextrakt Anthranilsäureamid-Gehalt (Summe Avenanthramide 3, 4 und 5) = 500 ppm in Glycerin | | | | |
| GS101100-B: Rekonstitut bestehend aus synthetischen Avenanthramiden 3, 4 und 5 Anthranilsäureamid-Gehalt (Summe synthetischer Avenanthramide 3, 4 und 5) = 500 ppm in Glycerin | | | | |

| | | %-Inhibition | | |
|---|---|---|---|---|
| **No.** | **Strukturformel** | **0,5 ppm** | **5 ppm** | **50 ppm** |
| GS101100-A | Haferstrohextrakt-Fraktion | 57,80 | 30,00 | 111,30 |
| GS101100-B | 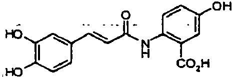 | | | |
| | 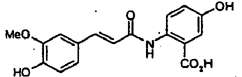 | | | |
| | 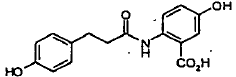 | 1,20 | 30,50 | 116,50 |

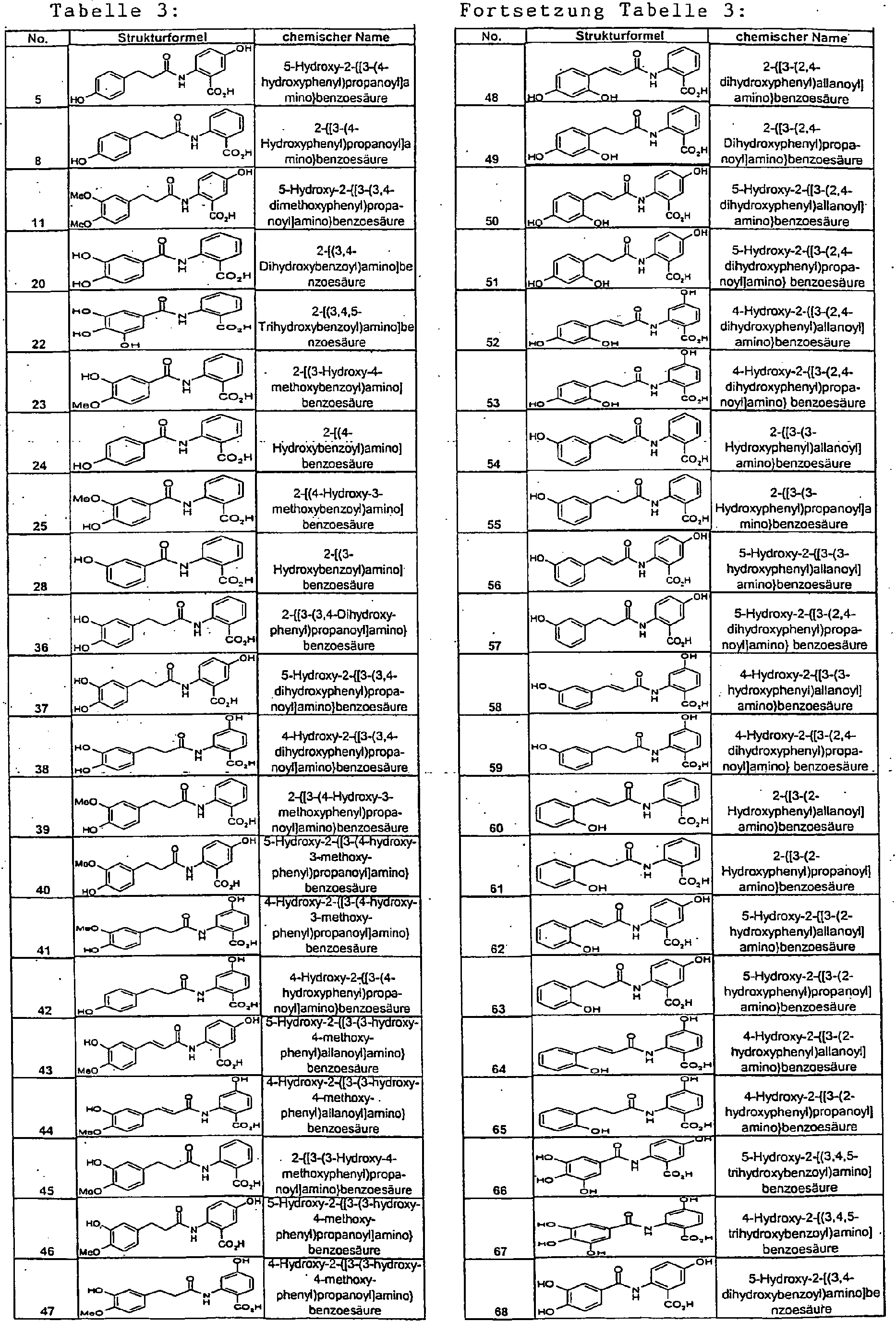

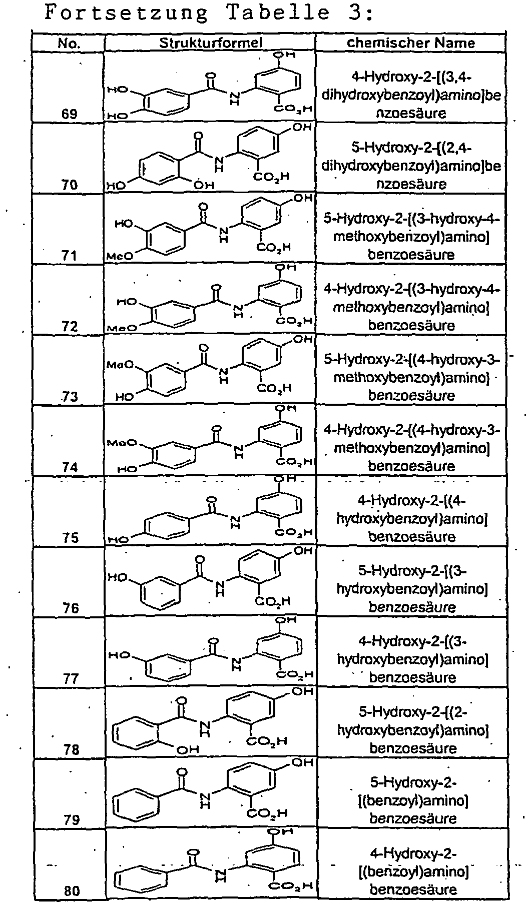

**Tabelle 4:**

| No. | Strukturformel | CAS-Nr. |
|---|---|---|
| 2 | 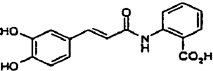 | 116764-16-0 |
| 3 | 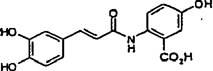 | 116764-15-9 |
| 4 | 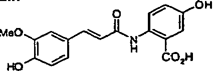 | 108605-69-2 |
| 6 | 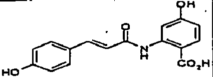 | 116764-17-1 |
| 7 | 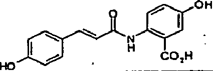 | 108605-70-5 |
| 9 | 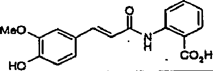 | 93755-77-2 |
| 10 | 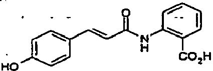 | 115610-36-1 |
| 12 | 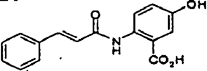 | 207742-91-4 |
| 13 | 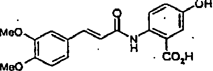 | 188545-62-2 |
| 21 | 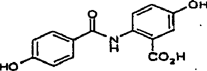 | 110846-17-8 |
| 26 | 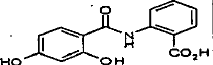 | 115610-37-2 |
| 27 | 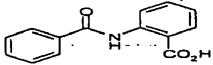 | 579-93-1 |
| 29 | 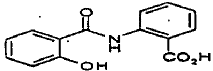 | 13316-98-8 |
| 30 | 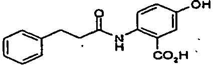 | 446029-17-0 |
| 31 | 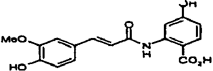 | 116764-18-2 |
| 32 | 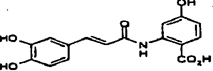 | 116764-19-3 |
| 33 | 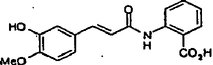 | 80530-43-4 |
| 34 | 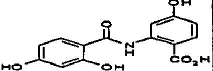 | 115610-38-3 |
| 35 | 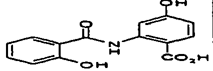 | 115610-40-7 |

## Patentansprüche

1. Kosmetische Verwendung einer Verbindung der Formel 1 oder einer Mischung aus zwei oder mehr unterschiedlichen Verbindungen der Formel 1, 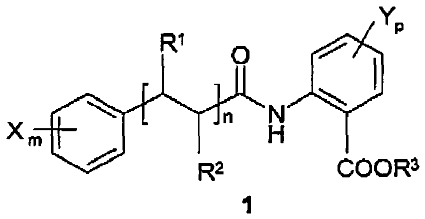 wobei für die Verbindung bzw, jede Verbindung der Mischung gilt
m = 0, 1, 2 oder 3,
p = 0, 1 oder 2,
n=1,
mit der Maßgabe, dass die Summe p + m > 0 ist,
wobei R¹ und R² jeweils H bedeuten;
wobei bei m = 1, 2 oder 3 jedes X, unabhängig von den anderen, OH, OAlkyl oder OAcyl bedeutet,
wobei bei p = 1 oder 2 jedes Y, unabhängig von den anderen, OH, OAlkyl OAcyl bedeutet,
mit der Maßgabe, dass mindestens einmal X oder Y aus der Gruppe ausgewählt ist, die aus OH und OAcyl besteht;
R³ = H oder Alkyl,
zur nicht therapeutischen Behandlung von oder Vorbeugung gegen Juckreiz (Pruritus), Hautrötung oder Quaddelbildung.

2. Verwendung einer Verbindung der Formel 1 oder einer Mischung aus zwei oder mehr unterschiedlichen Verbindungen der Formel 1, 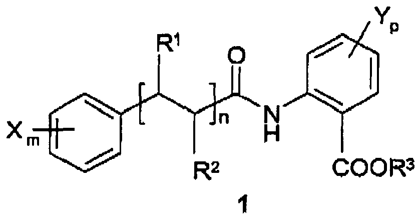 wobei für die Verbindung bzw, jede Verbindung der Mischung gilt:
m = 0, 1, 2 oder 3,
p = 0, 1 oder 2,
n = 1,
mit der Maßgabe, dass die Summe p + m > 0 ist.
wobei R¹ und R² jeweils H bedeuten;
wobei bei m = 1, 2 oder 3 jedes X, unabhängig von den anderen, OH, OAlkyl oder OAcyl bedeutet,
wobei bei p = 1 oder 2 jedes Y, unabhängig von den anderen, OH, OAlkyl OAcyl bedeutet,
mit der Maßgabe, dass mindestens einmal X oder Y aus der Gruppe ausgewählt ist, die aus OH und OAcyl besteht;
R³ = H oder Alkyl,
zur Herstellung eines Arzneimittels zur Behandlung von oder Vorbeugung gegen Juckreiz (Pruritus), Hautrötung, Quaddelbildung oder allergische Hautreaktionen.

3. Verwendung nach einem der Ansprüche 1 oder 2, wobei gilt:
m+p≥2
mit der Maßgabe, dass X und Y zusammen zumindest zweimal aus der Gruppe gewählt sind, die aus OH und OAcyl besteht.

4. Verwendung nach Anspruch 3, wobei die Verbindung der Formel 1 aus der Gruppe gewählt ist, die besteht aus:

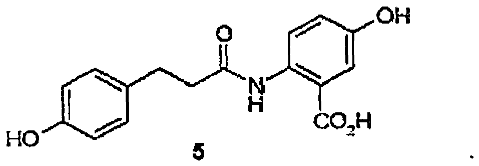

5. Verwendung nach einem der Ansprüche 1-4, wobei die Verbindung der Formel 1 oder die Mischung aus zwei oder mehr unterschiedlichen Verbindungen der Formel 1 als Bestandteil eines gegebenenfalls nachbehandelten Pflanzenextraktes vorliegt.

6. Verwendung nach Anspruch 5, wobei der gegebenenfalls nachbehandelte Pflanzenextrakt ein Extrakt aus Pflanzen der Gattung Avena. Dianthus, Silene oder Melandrium ist.

7. Verwendung nach Anspruch 5 oder 6, wobei der Pflanzenextrakt so nachbehandelt ist, dass im Vergleich mit dem nicht nachbehandelten Extrakt der Anteil an zumindest einer Verbindung der Formel 1 relativ zu dem Anteil sonstiger extrahierter Verbindungen erhöht ist.

8. Verwendung nach einem der Ansprüche 1-4, wobei eine isolierte und aufgereinigte Verbindung der Formel 1 oder eine isolierte und aufgereinigte Mischung aus zwei oder mehr unterschiedlichen Verbindungen der Formel 1 eingesetzt wird.

9. Mittel zur Behandlung von oder Vorbeugung gegen Juckreiz (Pruritus), Hautrötung, Quaddelbildung und/oder allergische Hautreaktionen, enthaltend eine Verbindung der Formel 1, 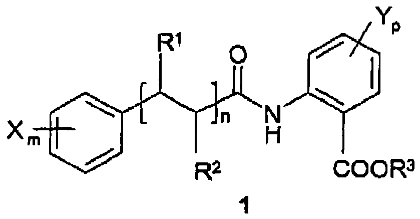 wobei gilt
m = 0, 1, 2 oder 3,
p = 0, 1 oder 2,
n=1
mit der Maßgabe, dass die Summe p + m > 0 ist,
wobei R¹ und R² jeweils H bedeuten;
wobei bei m = 1, 2 oder 3 jedes X, unabhängig von den anderen, OH, OAlkyl oder OAcyl bedeutet,
wobei bei p = 1 oder 2 jedes Y, unabhängig von den anderen, OH, OAlkyl oder OAcyl bedeutet,
mit der Maßgabe, dass mindestens einmal X oder Y aus der Gruppe gewählt ist, die aus OH und OAcyl besteht;
R³ = H oder Alkyl,
wobei die Verbindung der Formel aus der Gruppe gewählt ist, die besteht aus:

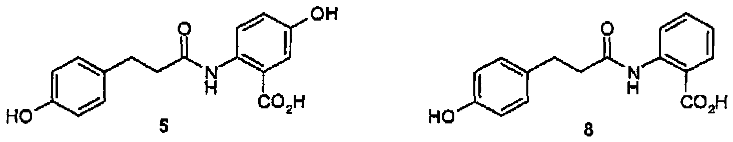

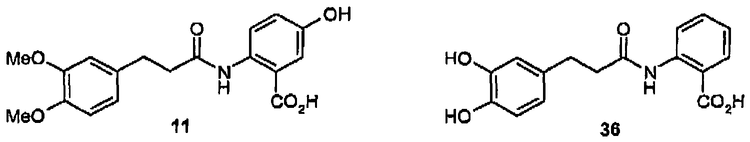

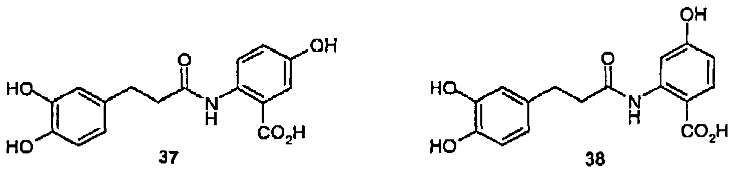

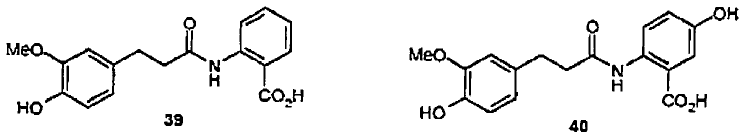

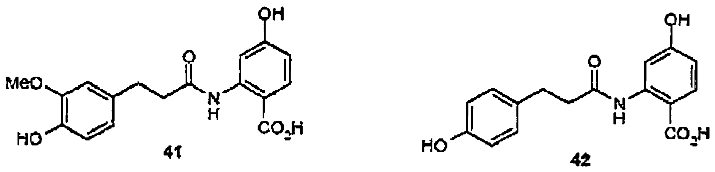

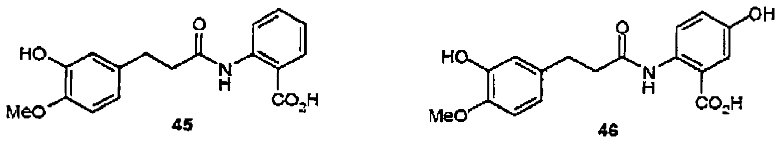

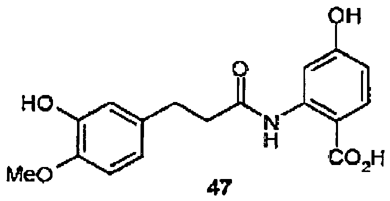

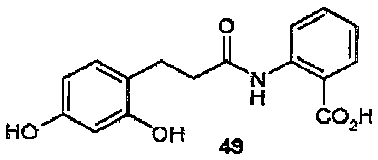

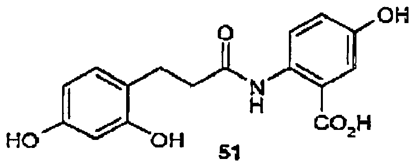

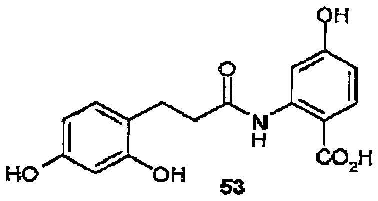

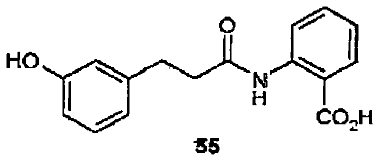

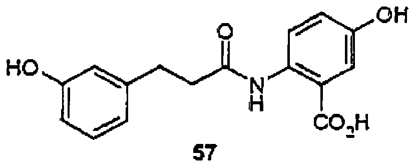

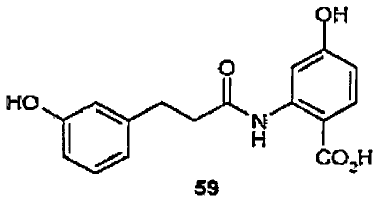

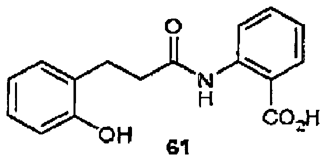

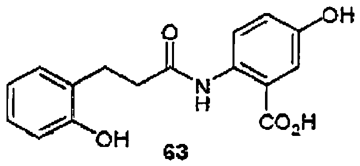

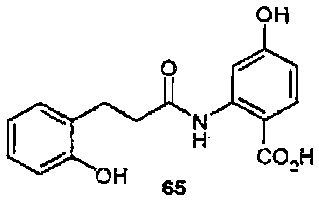

10. Verbindung der Formel 1, 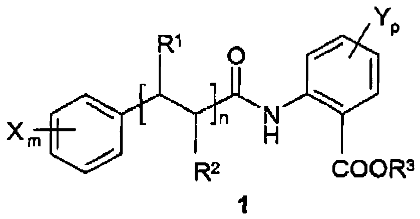 wobei gilt:
m = 0, 1, 2 oder 3,
p = 0, 1 oder 2,
n = 1
mit der Maßgabe, dass die Summe p + m > 0 ist,
wobei R¹ und R² jeweils H bedeuten;
wobei bei m = 1, 2 oder 3 jedes X. unabhängig von den anderen, OH, OAlkyl oder OAcyl bedeutet,
wobei bei p = 1 oder 2 jedes Y, unabhängig von den anderen, OH, OAlkyl oder OAcyl bedeutet,
mit der Maßgabe, dass mindestens einmal X oder Y aus der Gruppe gewählt ist, die aus OH und OAcyl besteht;
R³ = H oder Alkyl,
wobei die Verbindung der Formel aus der Gruppe gewählt ist, die besteht aus:

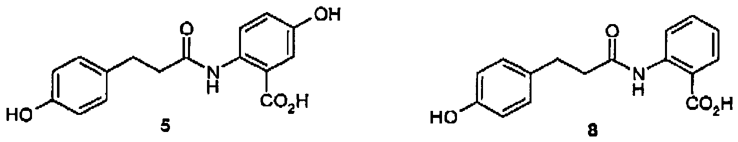

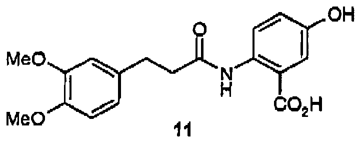

## Claims

1. Cosmetic use of a compound of formula 1 or a mixture of two or more different compounds of formula 1, 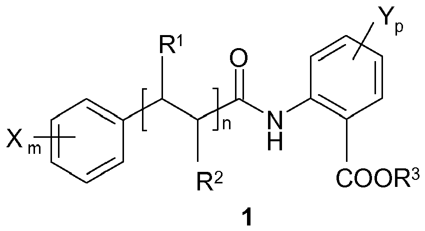 wherein for the compound or each compound of the mixture, respectively, the following applies:
m = 0, 1, 2 or 3,
p = 0, 1 or 2,
n = 1,
with the proviso that the sum p + m > 0,
wherein R¹ and R² each mean H;
wherein in case of m = 1, 2 or 3 each X, independent of the others, means OH, OAlkyl or OAcyl,
wherein in case of p = 1 or 2 each Y, independent of the others, means OH, OAlkyl or OAcyl,
with the proviso that X or Y are at least once selected from the group which consists of OH and OAcyl;
R³ = H or alkyl,
for the non-therapeutic treatment or prevention of itchy skin (pruritus), reddening of the skin or the formation of urtica.

2. Use of a compound of formula 1 or a mixture of two or more different compounds of formula 1, 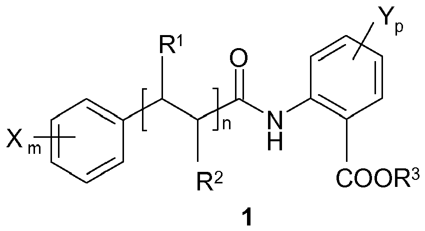 wherein for the compound or each compound of the mixture, respectively, the following applies:
m = 0, 1, 2 or 3,
p = 0, 1 or 2,
n = 1,
with the proviso that the sum p + m > 0,
wherein R¹ and R² each mean H;
wherein in case of m = 1, 2 or 3 each X, independent of the others, means OH, OAlkyl or OAcyl,
wherein in case of p = 1 or 2 each Y, independent of the others, means OH, OAlkyl or OAcyl,
with the proviso that X or Y are at least once selected from the group which consists of OH and OAcyl;
R³ = H or alkyl,
for the production of a medicament for the treatment or prevention of itchy skin (pruritus), reddening of the skin, the formation of urtica or allergic skin reactions.

3. Use according to one of claims 1 or 2, wherein the following applies:
m + p ≥ 2
with the proviso that X and Y together are at least twice selected from the group which consists of OH and OAcyl.

4. Use according to claim 3, wherein the compound of formula 1 is selected from the group which consists of:

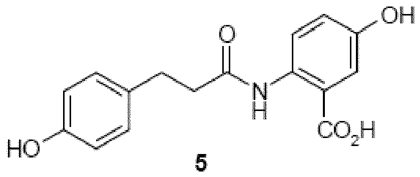

5. Use according to one of the claims 1-4, wherein the compound of formula 1 or the mixture of two or more different compounds of formula 1 are present as a component of a plant extract which has optionally been given an after-treatment.

6. Use according to claim 5, wherein the plant extract, which has optionally been given an after-treatment, is an extract from plants of the species avena, dianthus, silene or melandrium.

7. Use according to claim 5 or 6, wherein the plant extract has been given an after-treatment, so that in comparison with the extract, which has nor been given an after-treatment, the portion of at least one compound of formula 1 is increased relative to the portion of other extracted compounds.

8. Use according to one of the claims 1-4, wherein one isolated and purified compound of formula 1 or one isolated and purified mixture of two or more different compounds of formula 1 is used.

9. Means for the treatment or prevention of itchy skin (pruritus), reddening of the skin, formation of urtica and/or allergic skin reactions, comprising a compound of formula 1, 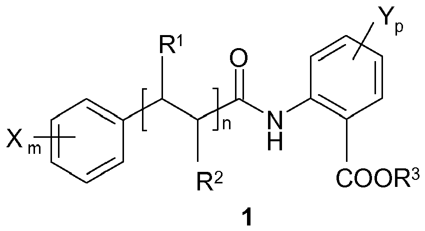 wherein the following applies:
m = 0, 1, 2 or 3,
p = 0, 1 or 2,
n = 1
with the proviso that the sum p + m > 0,
wherein R¹ and R² each mean H;
wherein in case of m = 1, 2 or 3 each X, independent of the others, means OH, OAlkyl or OAcyl,
wherein in case of p = 1 or 2 each Y, independent of the others, means OH, OAlkyl or OAcyl,
with the proviso that X or Y are at least once selected from the group which consists of OH and OAcyl;
R³ = H or alkyl,
wherein the compound of the formula is selected from the group which consists of:

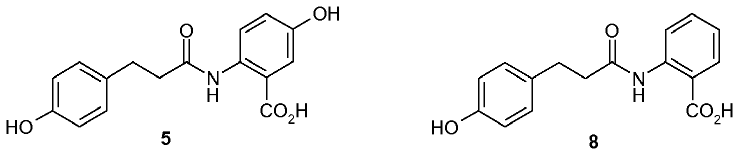

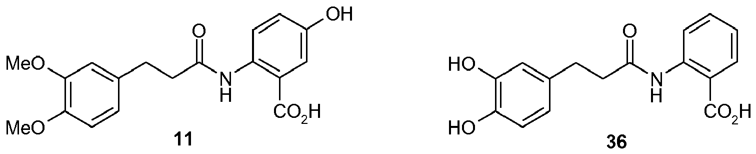

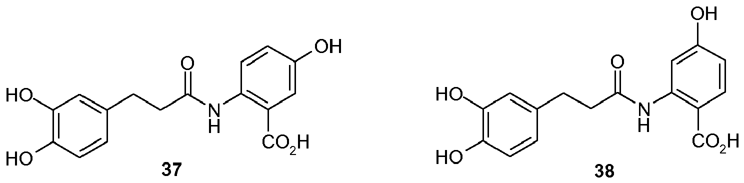

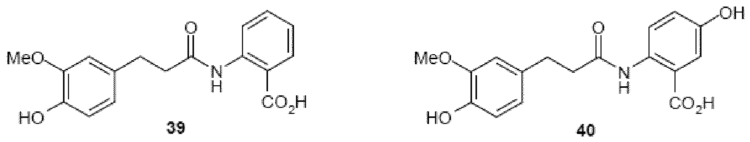

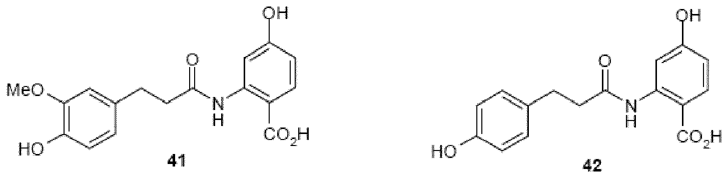

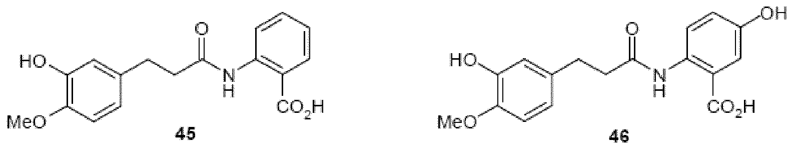

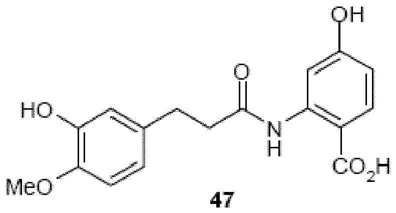

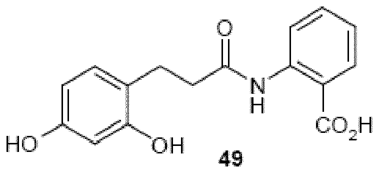

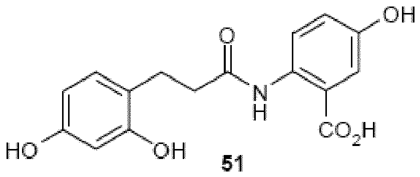

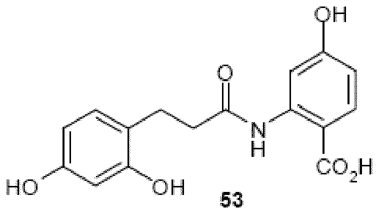

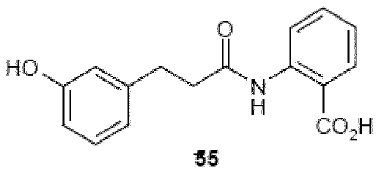

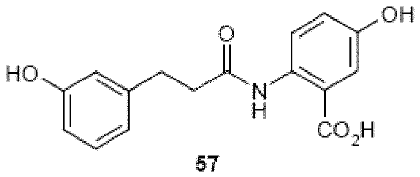

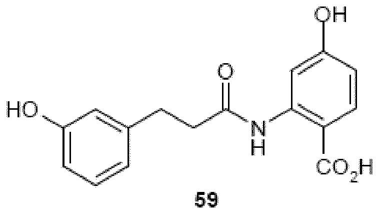

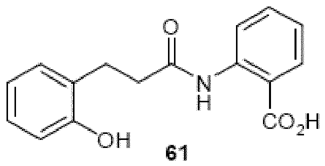

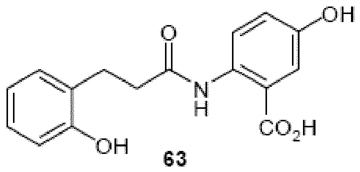

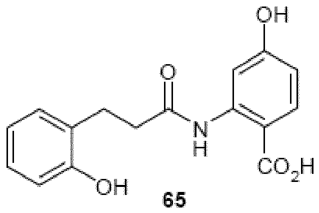

10. Compound of formula 1, 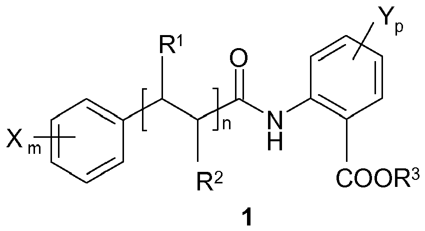 wherein the following applies:
m = 0, 1, 2 or 3,
p = 0, 1 or 2,
n = 1
with the proviso that the sum p + m > 0,
wherein R¹ and R² each mean H;
wherein in case of m = 1, 2 or 3 each X, independent of the others, means OH, OAlkyl or OAcyl,
wherein in case of p = 1 or 2 each Y, independent of the others, means OH, OAlkyl or OAcyl,
with the proviso that X or Y are at least once selected from the group which consists of OH and OAcyl;
R³ = H or alkyl,
wherein the compound of the formula is selected from the group which consists of:

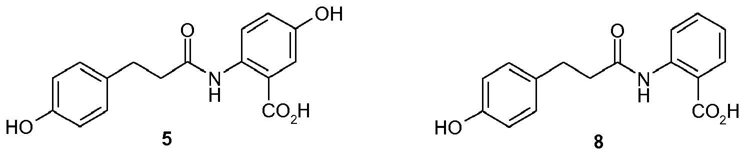

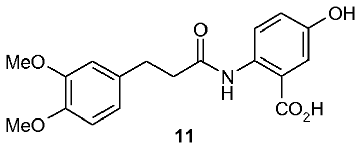

## Revendications

1. Utilisation cosmétique d'un composé de la formule 1 ou d'un mélange de deux composés différents ou plus de la formule 1, 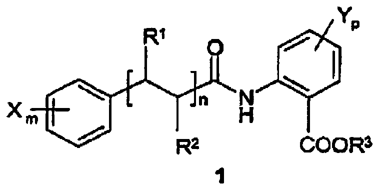 où, pour le composé ou bien chaque composé du mélange, vaut :
m = 0, 1, 2 ou 3,
p = 0, 1 ou 2,
n = 1,
sous réserve que la somme p + m est > 0,
où R¹ et R² signifient respectivement H ;
où, dans le cas de m = 1, 2 ou 3, chaque X, indépendamment des autres, signifie OH, Oalkyle ou Oacyle,
où, dans le cas de p = 1 ou 2, chaque Y, indépendamment des autres, signifie OH, Oalkyle ou Oacyle,
sous réserve que X ou Y est choisi au moins une fois dans le groupe qui se compose d'OH et d'Oacyle;
R³ = H ou alkyle,
pour le traitement non-thérapeutique de ou la prévention contre le prurit (pruritus), la rougeur de la peau ou la formation de papules.

2. Utilisation d'un composé de la formule 1 ou d'un mélange de deux composés différents ou plus de la formule 1, 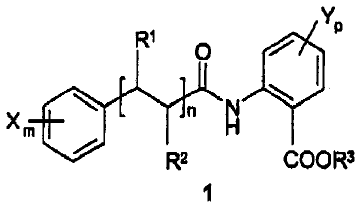 où, pour le composé ou bien chaque composé du mélange, vaut :
m = 0, 1, 2 ou 3,
p = 0, 1 ou 2,
n = 1,
sous réserve que la somme p + m est > 0,
où R¹ et R² signifient respectivement H ;
où, dans le cas de m = 1, 2 ou 3, chaque X, indépendamment des autres, signifie OH, Oalkyle ou Oacyle,
où, dans le cas de p = 1 ou 2, chaque Y, indépendamment des autres, signifie OH, Oalkyle ou Oacyle,
sous réserve que X ou Y est choisi au moins une fois dans le groupe qui se compose d'OH et d'Oacyle ;
R³ = H ou alkyle,
pour la préparation d'un médicament destiné au traitement de ou la prévention contre le prurit (pruritus), la rougeur de la peau, la formation de papules ou des réactions allergiques de la peau.

3. Utilisation selon l'une quelconque des revendications 1 ou 2, dans laquelle vaut :
m + p ≥ 2
sous réserve que X et Y sont choisis ensemble au moins deux fois dans le groupe qui se compose d'OH et d'Oacyle.

4. Utilisation selon la revendication 3, dans laquelle le composé de la formule 1 est choisi dans le groupe qui se compose de :

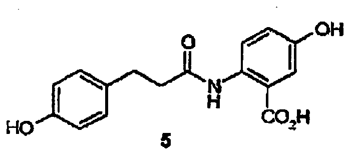

5. Utilisation selon l'une quelconque des revendications 1 à 4, dans laquelle le composé de la formule 1 ou le mélange de deux composés différents ou plus de la formule 1 se présente en tant que composant d'un extrait végétal le cas échéant traité ultérieurement.

6. Utilisation selon la revendication 5, dans laquelle ledit extrait végétal le cas échéant traité ultérieurement est un extrait de plantes du genre Avena, Dianthus, Silène ou Melandrium.

7. Utilisation selon la revendication 5 ou 6, dans laquelle ledit extrait végétal est traité ultérieurement de telle sorte que, en comparaison de l'extrait non traité ultérieurement, la part d'au moins un composé de la formule 1 par rapport à la part d'autres composés extraits est élevée.

8. Utilisation selon l'une quelconque des revendications 1 à 4, dans laquelle est utilisé un composé isolé et purifié de la formule 1 ou un mélange isolé et purifié de deux composés différents ou plus de la formule 1.

9. Agent pour le traitement de ou la prévention contre le prurit (pruritus), la rougeur de la peau, la formation de papules et/ou des réactions allergiques de la peau, contenant un composé de la formule 1, 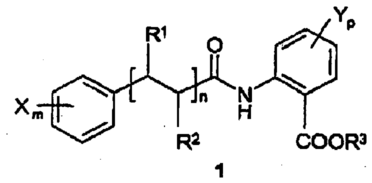 où vaut :
m = 0, 1, 2 ou 3,
p = 0, 1 ou 2,
n = 1,
sous réserve que la somme p + m est > 0,
où R¹ et R² signifient respectivement H ;
où, dans le cas de m = 1, 2 ou 3, chaque X, indépendamment des autres, signifie OH, Oalkyle ou Oacyle,
où, dans le cas de p = 1 ou 2, chaque Y, indépendamment des autres, signifie OH, Oalkyle ou Oacyle,
sous réserve que X ou Y est choisi au moins une fois dans le groupe qui se compose d'OH et d'Oacyle ;
R³ = H ou alkyle,
le composé de la formule étant choisi dans le groupe qui se compose de :

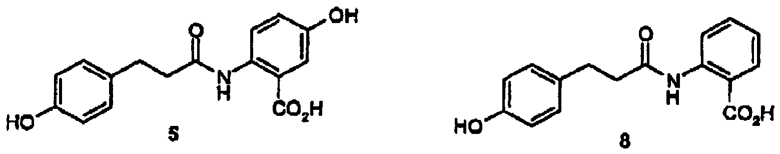

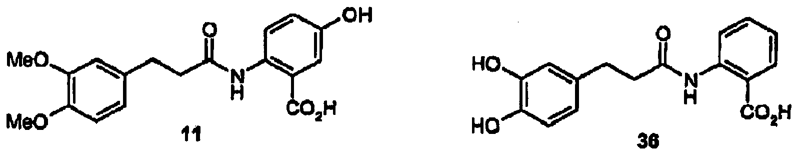

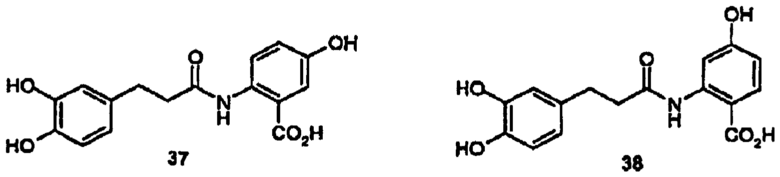

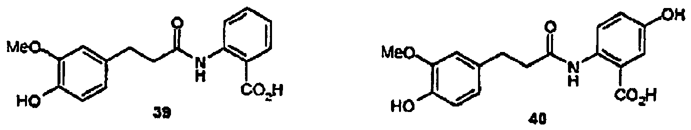

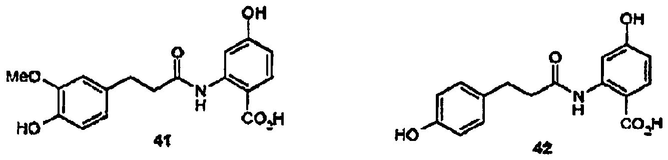

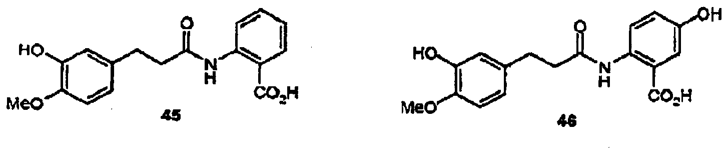

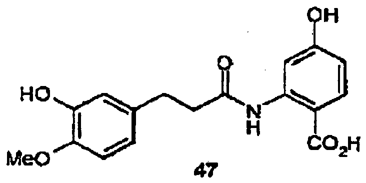

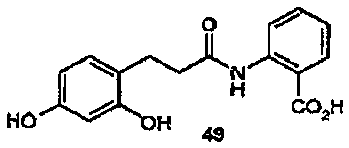

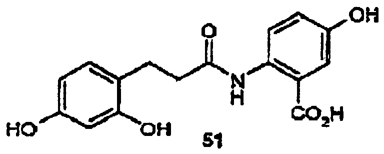

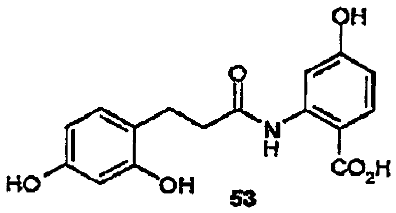

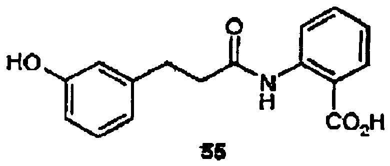

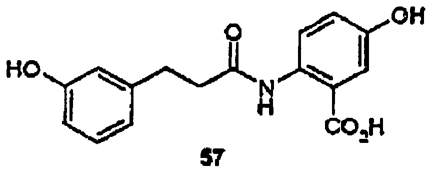

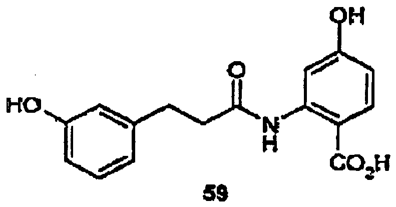

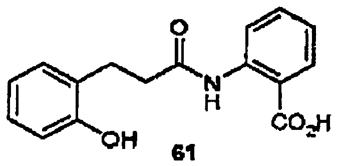

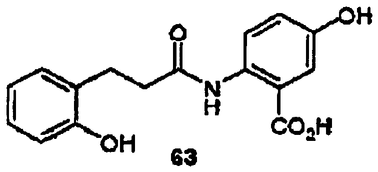

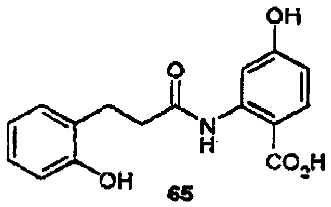

10. Composé de la formule 1, 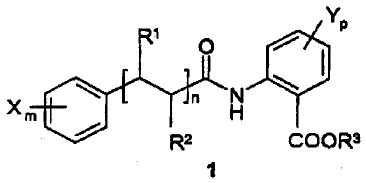 où vaut :
m = 0, 1, 2 ou 3,
p = 0, 1 ou 2,
n = 1,
sous réserve que la somme p + m est > 0,
où R¹ et R² signifient respectivement H ;
où, dans le cas de m = 1, 2 ou 3, chaque X, indépendamment des autres, signifie OH, Oalkyle ou Oacyle,
où, dans le cas de p = 1 ou 2, chaque Y, indépendamment des autres, signifie OH, Oalkyle ou Oacyle,
sous réserve que X ou Y est choisi au moins une fois dans le groupe qui se compose d'OH et d'Oacyle ;
R³ = H ou alkyle,
le composé de la formule étant choisi dans le groupe qui se compose de :
